# EUROPEAN PATENT SPECIFICATION

(11) **EP 2 010 220 B1**
(45) Date of publication and mention of the grant of the patent: **16.02.2022**
(21) Application number: 07754571.3
(22) Date of filing: 03.04.2007
(51) Int. Cl.: A61K 31/737, A61K 47/02, A61K 47/14, A61K 47/18, A61K 9/19, A61P 7/02, A61P 9/10, A61P 9/14, A61P 13/10, A61P 13/12, A61P 17/02, A61P 19/02, A61P 25/00, A61P 31/18, A61K 9/00

(54) **STABILIZED PENTOSAN POLYSULFATE (PPS) FORMULATIONS**
STABILISIERTE PENTOSAN-POLYSULFAT (PPS)-FORMULIERUNGEN
FORMULATIONS DE POLYSULFATE DE PENTOSANE STABILISÉ (PPS)

(30) Priority: 03.04.2006 US 788052 P
(43) Date of publication of application: 07.01.2009
(73) Proprietor: Ceva Animal Health Pty Ltd, Glenorie, NSW 2157 (AU)
(72) Inventor: ELLINGHUYSEN, Jerry, A., Loveland, CO 80538 (US); FILBURN, Charles, Forest Hill, MD 21050 (US); GRIFFIN, David, Belair, MD 21014 (US); HENDERSON, Todd, R., Jerrettsville, MD 21084 (US)
(74) Representative: Cabinet Becker et Associés
(86) International application number: PCT/US2007/008066
(87) International publication number: WO 2007/123800

(56) References cited:
- US-A- 5 470 840
- US-A- 5 643 892
- US-B1- 6 255 295
- US-B2- 6 828 309
- SCHIRM BERNHARD ET AL: "Improvements in pentosan polysulfate sodium quality assurance using fingerprint electropherograms", ELECTROPHORESIS, vol. 22, no. 6, April 2001 (2001-04), pages 1150-1162, XP002694171, ISSN: 0173-0835
- DEGENHARDT MATTHIAS ET AL: "Studies on the structural variations of pentosan polysulfate sodium (NaPPS) from different sources by capillary electrophoresis", ARCHIV DER PHARMAZIE (WEINHEIM), vol. 334, no. 1, January 2001 (2001-01), pages 27-29, XP002694172, ISSN: 0365-6233
- PROCHAZKA S ET AL: "Optimisation for the separation of the oligosaccharide, sodium Pentosan Polysulfate by reverse polarity capillary zone electrophoresis using a central composite design.", JOURNAL OF PHARMACEUTICAL AND BIOMEDICAL ANALYSIS, vol. 31, no. 1, 5 February 2003 (2003-02-05), pages 133-141, XP002694173, ISSN: 0731-7085
- KLOCKOW A. ET AL.: 'Detection of Carbohydrates' JOURNAL OF CHROMATOGRAPHY vol. 720, 1996, pages 355 - 360, XP008130851

## Description

### Field of the invention

The present invention is directed to pentosan polysulfate (PPS) formulations. More particularly, the present invention is directed to various PPS formulations having improved resistance to degradation and discoloration and improved stability, even after sterilization. The present invention is also directed to methods of using these formulations as an anticoagulant and/or to treat arthritis and other conditions, such as interstitial cystitis, in mammals.

### Related Art

Pentosan polysulfate (PPS) is a semi-synthetic, polysulfated oligosaccharide comprising a mixture of multiply charged anionic polysaccharides. PPS is produced by chemical sulfation of polysaccharides (e.g., xylan) obtained from woody plants, for example beechwood trees. The resulting product typically contains approximately 15-17% sulfur in the form of approximately 1.5-1.9 covalently bound sulfate groups per sugar residue in a mixture of polydisperse polymeric molecules estimated to be approximately 4,000-10,000 in molecular weight. PPS consists of sulfated, linear polysaccharides of about 12 to 30 1-4 conjugated *beta*-D-xylopyranose units (Mᵣ = approx. 4,000 - approx. 10,000), which has a D-gluconic acid at approximately every tenth unit. PPS is typically semi-synthetically manufactured from phytogenic substances or may be obtained from microorganisms.

PPS is most commonly used as an oral formulation to treat interstitial cystitis in humans and as an injectable drug to treat osteoarthritis in companion animals (Fuller, Ghosh et al., "Plasma and synovial fluid concentrations of calcium pentosan polysulfate achieved in the horse following intramuscular injection," Equine Veterinary Journal (2002)). The compound PPS may also be used as an anticoagulant, preventing the formation of blood clots. It has also been used for treatment of hematomes, hemorrhoids, frostbites, burns, and multiparameter illnesses such as thrombosis and atherosclerosis. US 6,828,309 discloses the use of liquid formulations comprising PPS to treat certain conditions of the prostate, such as benign prostatic hyperplasia, chronic prostatitis, prostadynia, and irritative bladder conditions.

While uses of PPS are becoming more widespread, a fundamental problem persists in developing a product that will not degrade or discolor under certain conditions of use, and can be used under a variety of conditions. Injectable PPS formulations tend to degrade and discolor over time, limiting their shelf life. Moreover, some formulations of PPS have been observed to turn brownish in color over time. In addition, customary characterization methods for defining PPS formulations, which are an important element in quality control, are generally considered to be inadequate. Thus, at present it remains unclear what specific characteristics of PPS formulations actually relate to stability and clinical effectiveness.

The polydisperse nature of PPS necessitates a method for quality control that detects variations in composition relating to size, degree of sulfation, or partial degradation. Standard methods for analysis such as thin layer chromatography, size exclusion chromatography (Maffrand, Herbert, et al., "Experimental and Clinical Pharmacology of Pentosan Polysulfate," Seminars in Thrombosis and Hemostatis, Volume 17, Supplement 2, 1991), or spectroscopy have not been shown to resolve these questions. Gel chromatography has been used successfully to test for the presence of sodium sulfate and sodium acetate. However, drawbacks of this method include long analysis times and poor efficiency in the separation of the composition into its component parts.

Analysis by capillary electrophoresis (CE) has been used to generate a profile of peaks that appears to be capable of distinguishing a range of PPS molecules, including oligosaccharides differing at least in size (Degenhardt, Benend et al., "Quality control of pentosane polysulfate by capillary zone electrophoresis using indirect detection," Journal of Chromatography A 817 (1998); Degenhardt, Ghosh et al., "Studies on the Structural Variations of Pentosan Polysulfate Sodium (NaPPS) from Different Sources by Capillary Electrophoresis," Arch. Pharm. Pharm. Med. Chem. (2001)). One problem experienced with the CE method lies in the reproducibility of the migration times of the peaks. This problem has been partially resolved by resorting to a method of computerized pattern recognition and realignment of fingerprint profiles (Schirm, Benend et al., "Improvements in pentosan polysulfate sodium quality assurance using fingerprint electropherograms," Electrophoresis 2001). Without using this enhancement, comparisons were made of fingerprints obtained by analyzing and comparing one source of PPS to the other two in separate studies (Degenhardt et al., 1998, 2001). Degenhardt showed that the three sources differed in levels of shorter oligosaccharides peaks, with the suggestion being made that some of the heterogeneity may reflect non-PPS species (Degenhardt et al., 2001).

However, these articles did not describe a method that could be used to assess the characteristics of formulations that might degrade or the effects of storage after production of a given batch.

There is thus a need in the art for formulations of pentosan polysulfate that resist degradation and discoloration and a parallel need for testing methods that allow for routine and accurate characterization of such PPS formulations. Various embodiments of the present invention provide such formulations and methods of verifying the purity of the formulations.

### SUMMARY OF THE INVENTION

The invention provides various stable formulations comprising PPS. The subject-matter of the present invention is such as defined in claims 1-11. In one embodiment, the formulations are stable without refrigeration in a solution having a pH in the range of about 4 to about 8.. In another embodiment, the formulations are stable without refrigeration in a solution having a pH in the range of about 7 to about 8. In some embodiments, the formulations are stable without refrigeration after terminal sterilization.

In some embodiments, the molecules of PPS in the formulation have a substantially symmetrical distribution of molecular weights. For example, a chromatogram showing the number of molecules having particular molecular weights, e.g., as indicated in an electropherogram of a sample of the formulation, may be a substantially smooth and substantially symmetrical bell-shaped curve. In some embodiments, the molecules of PPS in the formulation have a substantially symmetrical distribution of molecular weights after terminal sterilization.

In some embodiments, the formulations comprising PPS molecules have substantially homogeneous molecular weights. For instance, the PPS molecules may consist essentially of molecules of PPS having substantially homogeneous molecular weights. In one embodiment, the formulation maintains substantially similar or homogeneous molecular weights after terminal sterilization. According to some embodiments, the formulations have substantially similar molecular weights after terminal sterilization. For example, an electropherogram of the formulation may be characterized by a substantially smooth bell-shaped curve corresponding to the presence of PPS molecules. In some embodiments, the molecules of PPS may have molecular weights substantially ranging from about 4,700 to about 10,700 daltons.

In some embodiments, liquid formulations according to the present invention may comprise oligosaccharides consisting essentially of pentosan polysulfate, wherein the formulation is stable without refrigeration.

In another embodiment, the formulations of the present invention remain stable under various conditions. For example, the formulations may remain stable for six months. Some formulations may remain stable for up to one, two, three, or five years. In some embodiments, the formulation may remain stable at a temperature that is significantly higher than room temperature, e.g., for a period of time such as about twelve hours, about a day, about one or two weeks, about a month, about six months, or about a year.

The invention provides a stable formulation comprising PPS in a concentration of about 25 to about 500 mg/mL. In some embodiments, the formulation may comprise PPS in a concentration of about 100 to about 250 mg/mL. In some embodiments, a capillary electrophoresis analysis of the formulation at a concentration of about 1-5 mg/mL shows a substantially bell-shaped curve corresponding to the presence of PPS in a graph of change-in-absorption-versus-time. The bell-shaped curve has a first half corresponding to an earlier absorption and a second half corresponding to a later absorption. The two halves meet at a peak portion of the bell-shape. The area inside the bell shape is substantially greater than the total area defined by any peaks that define deviations in the first half of the substantially bell-shaped curve. In other embodiments, the height of the bell is substantially taller than the height of any peaks in the first half of the bell shape.

It is also disclosed herein various methods of detecting degradation products in a formulation comprising PPS. In one embodiment, the formulation is exposed to conditions of forced degradation. Then capillary electrophoresis is used to prepare an electropherogram on a sample of the formulation. A degradation peak is identified in the electropherogram. The degradation peak is substantially taller than the peak of the bell.

It is further disclosed a method of sterilizing a formulation comprising PPS. In one embodiment, a formulation comprising PPS is prepared and then sterilized, e.g., by terminal sterilization in an autoclave. Methods such as CE analysis are used to show stability characteristics of the sterilized formulation, e.g., by detecting one or more degradation products other than sulfate.

The disclosure also describes a method of detecting a degradation product in a formulation comprising PPS. A first capillary electrophoresis measurement is conducted on a sample of the formulation. Afterwards, the formulation is subjected to one or more conditions, such as heat, acid, base, or lapse of time. Then, a second capillary electrophoresis measurement on a sample of the formulation is conducted. The first and second measurements are compared, e.g., by comparing their corresponding electropherograms. The second measurement indicates the presence (or lack) of degradation products that were not shown in the first measurement.

In another embodiment, a method for detecting an indicium of stability of a pentosan polysulfate (PPS) formulation using capillary electrophoresis is described. A sample of the formulation is diluted to a concentration of about 1 mg/mL to about 5 mg/mL in water. An electropherogram for the formulation is prepared using capillary electrophoresis in a manner that satisfies a Peak Resolution Standard (as defined below), the electropherogram comprising a change-in-absorption-versus-time graph. A substantially bell-shaped portion of the electropherogram substantially defining a bell and corresponding to PPS is identified.

One indicium of stability of the formulation based on a size characteristic of the bell in comparison to a size characteristic of one or more peaks, wherein size characteristics are determined by valley-to-valley integration. Another indicium of stability may be that the area of the bell is at least about thirteen times greater than the total combined area of one or more secondary peaks that may appear on the generally bell-shaped portion of the curve corresponding to PPS. Another indicium of stability may be that the height of the bell is more than about three times greater than the height of any secondary peak that satisfies a Pentosan Homogeneity Standard. Another indicium of stability may be that the height of the bell is more than about four times greater than a third highest peak. In some embodiments, the formulation may be subject to a degradation-potentiating condition before preparing the sample, such as the passage of time, or a temperature significantly higher than room temperature. Analyzing the electropherogram may indicate whether the PPS satisfies a Pentosan Homogeneity Standard, as defined herein.

In another embodiment, a liquid formulation comprising pentosan polysulfate (PPS) is provided. A capillary electrophoresis analysis of the formulation at a sample concentration of about 1 mg/mL to about 5 mg/mL may show a substantially bell-shaped curve corresponding to the presence of PPS in a graph of change-in-absorption-versus-time. The bell-shaped curve may comprise a first portion corresponding to an earlier absorption and a second portion corresponding to a later absorption. The first portion and the second portion may be joined at a middle peak portion. In some embodiments, the area inside the substantially bell-shaped portion of the curve is more than about thirteen times greater than the total area defined by all distinct peaks that appear in the first portion of the substantially bell-shaped curve, wherein area is determined by valley-to-valley integration.

The disclosure further describes a quality control method for detecting an indicia of stability of a PPS formulation using capillary electrophoresis. A sample of the formulation with a concentration of about 1 mg/mL to about 5 mg/mL is diluted in water. An electropherogram for the formulation sample is prepared using capillary electrophoresis. The electropherogram comprises a change-in-absorption-versus-time graph. A substantially bell-shaped portion of the electropherogram substantially defines a bell and corresponds to PPS. The bell has a height and defines an area inside the bell. The bell has a first half corresponding to an earlier absorption and a second half corresponding to a later absorption. The first half comprises one or more peaks. Each peak has a height and area corresponding to its deviation from the bell. An indicium of stability of the formulation is determined as an element of quality control based on a comparison of a size characteristic of the bell and a size characteristic of the one or more peaks.

In yet another embodiment, a formulation of the invention comprises a stable, sterilized pentosan polysulfate formulation that is substantially free from brown impurities, including for example after terminal sterilization. Some formulations may be substantially free of degradation products of PPS, including for example after terminal sterilization.

### BRIEF DESCRIPTION OF THE DRAWINGS

FIG. 1 shows the basic chemical structure of pentosan polysulfate.
FIG. 2 shows a schematic of an exemplary high-performance capillary electrophoresis instrument.
FIG. 3 shows a cross-sectional view of an exemplary capillary 300 in a capillary electrophoresis instrument.
FIG. 4 shows an exemplary schematic of electrophoresis and, electroosmosis in a separation of anionic, neutral, and cationic analytes.
FIG. 5 shows an exemplary system for conducting electrophoresis measurements on sample formulations.
FIGS. 6A and 6B show an exemplary change-in-absorption-versus-time curve for an exemplary sample traveling through a capillary generated using indirect detection.
FIGS. 7-9 show electropherograms for PPS-containing substances that were not formulated according to the present invention.
FIGS. 10-13 show electropherograms for a commercially available formulation of PPS.
FIG. 14 shows an exemplary electropherogram of PPS raw material.
FIGS. 15A and 15B show electropherograms for a commercially available PPS-containing substance a substantial period of time after acquisition.
FIGS. 16A, 16B, 16C, and 16D show electropherograms of exemplary formulations of PPS in accordance with various embodiments of the invention.
FIGS. 17A, 17B, 18A, 18B, 19A, 19B, 20A, and 20B show electropherograms for exemplary formulations of PPS before and after sterilization, in accordance with various embodiments of the invention.
FIGS. 21, 22A, 22B, 23A, and 23B show electropherograms after forced degradation of an exemplary formulation according to the present invention.
FIG. 24 shows an electropherogram of a commercially available PPS-containing formulation after forced degradation.
FIGS. 25A, 25B, 26A, 26B, 27A, 27B, 28A, and 28B show electropherograms for sterilized and un-sterilized samples comprising PPS that were stored at different temperatures.
FIG. 29 shows an electropherogram of a perchlorate anion in a sample of sodium perchlorate.
FIGS. 30A and 30B show an electropherogram of a sample of pentosan in the absence and presence of added perchlorate.
FIGS. 31A and 31B shows an electropherogram of pentosan API diluted into water in the absence and presence of perchlorate.
FIG. 32 shows an electropherogram of the formate anion present in sodium formate.
FIGS. 33A and 33B show an electropherogram of pentosan API diluted in water in the absence and presence of formate.

### DETAILED DESCRIPTION OF EXEMPLARY EMBODIMENTS

Various embodiments disclosed herein are directed to formulations containing pentosan polysulfate according to the invention, methods of using such formulations, and methods for evaluating such formulations. The pentosan polysulfate formulations of the present invention may be used as an anticoagulant and/or to treat arthritis (e.g., osteoarthritis) or other conditions such as interstitial cystitis, transmissible spongiform encephalopathy (TSE), for example bovine spongiform encephalopathy (BSE), and immunodeficiency virus (such as HIV/AIDS or Feline Immunodeficiency Virus (FIV)) in mammals, such as humans, food-producing mammals, and companion mammals (such as feline, canine and equine). PPS may also be used to treat hematomes, hemorrhoids, frostbites, burns, and multiparameter illnesses such as thrombosis and atherosclerosis, including for example in such mammals.

Because PPS may be used under various conditions and, particularly in the veterinary context may be used in the field, there is a need for PPS formulations that resist or substantially resist degradation and/or discoloration under various conditions, such as sterilization, microbial challenge, storage over time, light and heat.

Moreover, because PPS may be ingested or injected into mammals, there is a further need for formulations that remain stable at a physiologic pH.

There is also a need for a reproducible method of quality control to assess batches of PPS, for example batches provided by a potential supplier. Preferably, the method should detect changes that may occur in the product following degradation.

Experiments were conducted to determine the efficacy of using capillary electrophoresis (CE) to evaluate various PPS formulations and changes in those formulations under various conditions. A capillary electrophoresis instrument was used to analyze PPS samples, and the results, as set forth in an electropherogram, were compared to the source described in the studies cited above. Based on these results, it was possible to determine whether CE has utility in detecting degradation. These experiments also enabled identification of various PPS formulations that have desirable characteristics, such as resistance to degradation.

The experiments were performed on a Beckman-Coulter PACE-MDQ capillary electrophoresis system using a method essentially the same as described by Degenhardt et al. (Degenhardt, Ghosh et al. 2001). These experiments demonstrate that a stabilized formulation of the present invention is different from that of an existing commercially available formulation (Cartrophen^{®}). Specifically, a stabilized formulation of the present invention does not contain appreciable levels of smaller oligosaccharides previously demonstrated to be present and characteristic of Cartrophen^{®}, which is one example of an injectable form of PPS currently available on the market (Degenhardt, Benend et al. 1998; Degenhardt, Ghosh et al. 2001). The Degenhardt, Benend et. al. 1998 and Degenhardt, Ghosh et al. 2001 articles, as well as the Schirm et al. 2001, Fuller et al. 1992, and Maffrand et. al. 1991 articles discussed above, are incorporated herein by reference in their entireties.

Embodiments of the present invention include a terminally sterilized PPS formulation that is suitable for administration to mammals. For example, in some embodiments, the formulation may be provided to mammals as a drug or dietary supplement. Other embodiments of the present disclosure include a method that distinguishes between various PPS formulations and detects degradation products, including degradation products other than sulfate. Furthermore, this method has enabled the identification of various PPS formulations that have desirable properties such as resistance to degradation.

As used in this application, the following terms shall have the following meanings:
"Degradation product" (also called decomposition product) means one or more molecules resulting from a chemical change in the substance brought about over time and/or by the action of internal or external factors, e.g., light, temperature, pH, or water, or by reaction with an excipient and/or the immediate container/closure system. The appearance of degradation products in a PPS formulation is characterized in part by an overall decrease in the area of a bell-shaped peak corresponding to PPS in an electropherogram, as measured before and after the appearance of the degradation product(s) or an increase in the number or area of secondary peaks appearing on the leading edge of the bell-shaped curve.

In some embodiments, an electropherogram can show the absence of degradation products in a formulation. For example, in an electropherogram of various formulations of the present invention, the area of the bell-shaped curve corresponding to PPS is at least about thirteen times greater than the total combined area of the one or more peaks, wherein the area is determined by valley-to-valley integration, e.g., manual valley-to-valley integration. In some embodiments, the height of the bell is more than about three times greater than the height of any peak that satisfies a Pentosan Homogeneity Standard, as defined below.

"Forced degradation," or "stress testing," involves exposing a substance to real or simulated conditions that are more severe than a typical environment. In some cases these conditions can be more severe than those used for accelerated tests on a substance. Such severe conditions may be a pH, temperature, or pressure that is significantly higher or lower than normal conditions. (Normal conditions may include a pH range of 6.5-7.5 at standard temperature and pressure, for example.) Forced degradation can also involve exposing a substance to other conditions or substances. Forced degradation is typically conducted to provide data on forced decomposition products and decomposition mechanisms relating to a substance, such as a drug substance. The severe conditions that may be encountered during distribution of a substance such as a drug product can be analyzed by stress testing definitive batches of the drug substance. Forced degradation studies can be used to establish the inherent stability characteristics of component molecules, such as the degradation pathways of the molecules, and forced degradation may lead to identification of degradation products and hence support the suitability of the proposed analytical procedures. The detailed nature of the studies will depend on the individual substance and type of drug product.

"Fragment" means an incomplete molecule, for example of PPS, with an irregular, non-repeating pattern of peaks visible on the electropherogram.

"Stable" in reference to a PPS formulation means that it maintains a substantially constant amount (e.g., substantially constant concentration) of sulfate and exhibits substantially no discoloration (e.g., substantially no brown discoloration), or the total area of all peaks migrating prior to the main pentosan peak of the bell-shaped curve must not exceed 5% of the total pentosan area. For instance, a "stable" PPS formulation is capable of having these characteristics over a shelf life, e.g., a shelf life of six months, or in some cases up to one, two or three years. A capillary electrophoresis analysis of a "stable" PPS formulation sample would show little or no visible degradation peaks.

"Terminal sterilization" means the process in which a formulation in its final form including all materials and containers is sterilized. This terminal sterilization process can be performed, for example, by moist heat with or without rapid cooling fluids, ethylene oxide or radiation. A substance that has undergone the process of terminal sterilization is said to be "terminally sterilized."

It should be appreciated that PPS is often formulated as a salt, such as sodium PPS, calcium PPS, or potassium PPS, for example. Pentosan may be obtained naturally from plants, microorganisms, or synthesized. Accordingly, references to PPS throughout this application may refer to PPS as well as to the various salts thereof, as appropriate whether obtained naturally, synthetically or semi-synthetically.

Various formulations of the present invention may have one or more beneficial properties such as resistance to degradation. Such formulations in accordance with the present invention comprise PPS or a salt thereof and one or more of the following components: one or more buffers, such as sodium bisulfite, sodium citrate, and/or citric acid; one or more chelators or chelating agents, such as EDTA; one or more preservatives; one or more antimicrobial agents, such as methyl paraben; one or more antioxidants; and any other suitable excipients. In some formulations according to various embodiments of the present invention, PPS may be combined with one or more of the above components as well as aminosugar and/or hyaluronic acid.

Exemplary chelators that may be included in various formulations of the present invention include, for example, ethylenediaminetetraacetic acid (EDTA), diethylenetriaminepentaacetate (DPTA), sodium EDTA, and other known chelating agents.

Exemplary buffers that may be included in various formulations of the present invention include, for example, citrate, sodium hydroxide/levulinic acid, acetate, bicarbonate, bisulfite, sodium hydroxide/glycine, phosphate, and other known buffers.

Exemplary antioxidants that may be included in various formulations of the present invention include, for example, acetone, sodium bisulfite, metabisulfite sodium, and other known preservatives.

Exemplary antimicrobial agents that may be included in various formulations of the present invention include, for example, methyl or propyl parabens, benzyl alcohol, and other known antimicrobial agents.

Exemplary aminosugars that may be included in various formulations of the present invention include, for example, glucosamine hydrochloride, galactosamine, glucosamine sulfate, glucosamine phosphate, N-acetyl glucosamine, mannosamine, and fragments, salts, and mixtures thereof. In addition, the term aminosugar is also used herein to encompass aminosugars that may have been chemically modified yet retain their function. Such chemical modifications include but are not limited to esterification, sulfation, polysulfation, acetylation, and methylation. Moreover, it is contemplated that the term aminosugar can extend to any composition of matter that is insubstantially different from the aminosugars described above.

Various formulations may also comprise hyaluronic acid. Hyaluronic acid is a non-sulfated glycosaminoglycan. Hyaluronic acid may be derived from an animal (e.g. extracted from rooster combs or bovine vitreous humor) or from a microorganism (e.g. bacterial or yeast fermentation), for example.

Various formulations in accordance with the present invention or disclosed herein may comprise one or more of the foregoing components in any suitable concentration or amount. PPS is present in a concentration of about 25 mg/mL to about 500 mg/mL, or more preferably about 250 mg/mL. In another example, PPS may be present in a total amount of about 10 mg to about 5 g. Buffers, for example, including sodium citrate, citric acid, or other buffers, may be present in concentrations such as about 1 to about 100 mM. For example, in some embodiments, a buffer such as sodium citrate may have concentrations in the formulation of about 50 mM (14.7 mg/mL), or a buffer such as citric acid may comprise about 55 mM (about 10.5 mg/mL). EDTA may be present in concentrations such as about 0.01% to about 0.5% w/v, 0.1 mM to about 1 mM, about 0.25 mg/mL, or more preferably about 0.25% w/v. Chelators may be present in concentrations such as about 0.1 to about 1 mM. Preservatives may be present in concentrations such as about 0.1% to about 1%. Antioxidants may be present in concentrations such as about 0.1 to 10 mM. According to the invention, antioxidants are present in concentrations of about 0.02% w/v to about 5% w/v. Excipients (e.g., pharmaceutical excipients) may be present in any suitable concentration, e.g., concentrations of about 1 to about 90%.

In other embodiments of the invention, various formulations may comprise one or more of these components in any suitable concentration or amount. For example, PPS may be present in a concentration of about 25 mg/mL to about 500 mg/mL, or more preferably about 250 mg/mL. Buffers may be present in concentrations such as of about 0.005% to about 5% w/v. As disclosed herein, sodium bisulfite may be present in concentrations such as about 0.02% to about 1% w/v, 10 mg/mL, or more preferably about 1% w/v. (When added to formulation of the present invention, sodium metabisulfite can convert to sulfur dioxide and sodium bisulfite. In embodiments, between about 25% and almost all of the metabisulfite may, upon addition to formulations of the present invention, convert to sulfur dioxide and sodium bisulfite.)

EDTA may be present in concentrations such as about 0.01% to about 0.5% w/v, 0.1 mM to about 1 mM, about 0.25 mg/mL, or more preferably about 0.25% w/v. Sodium citrate may be present in concentrations such as about 0.1 to about 4% w/v, or more preferably about 1.47% w/v. Citric acid may be present in concentrations such as about 0.5% to about 2% w/v, or more preferably about 1.05% w/v. Antimicrobial agents such as methyl paraben may be present in concentrations such as about 0.05% to about 0.2% w/v, or about 1 mg/mL, or more preferably about 0.1% w/v.

The formulations of the present invention are a liquid form and preferably are formulated as an aqueous solution. The formulation is in a solution having a pH of about 4 to about 8. In some embodiments, the formulation may have a pH of about 7 to about 8.

It should be appreciated by those of ordinary skill in the art that the formulations of the present invention may be lyophilized to create a lyophilized dosage form, using techniques apparent to one of ordinary skill in the art in light of this specification. In addition, lyophilized dosage forms may be formulated to comprise, after reconstitution, a dosage form of any of the formulations described herein.

An exemplary formulation may comprise one or more of the following: PPS in a concentration of about 25 to about 500 mg/mL; metabisulfite or bisulfite (e.g., sodium bisulfite) in a concentration of about 0.05% w/v to 5% w/v; one or more chelators in a concentration of about 0.01% w/v to about 0.5% w/v; one or more buffers in a concentration of about 0.005% w/v to about 5% w/v; one or more antioxidants in a concentration of about 0.02% w/v to about 1% w/v; one or more antimicrobial agents in a concentration of about 0.05% w/v to about 0.2% w/v hyaluronic acid; and glucosamine.

In some embodiments, sodium bisulfite may be present in concentrations such as about 10 mg/mL. EDTA may be present in concentrations such as about 0.25 mg/mL. Sodium citrate may be present in concentrations such as about 14.7 mg/mL. Citric acid may be present in concentrations such as about 10.5 mg/mL. Methyl paraben may be present in concentrations such as about 1 mg/mL.

An exemplary embodiment may comprise an injectable dosage form comprising pentosan polysulfate (PPS) at a concentration of about 250 mg/mL; sodium bisulfite in a concentration of up to about 20 mg/mL; and EDTA at a concentration of about 0.25 mg/mL. The formulation may be stable in a pH range of about 6 to about 7. The formulation may be formulated in any dosage form, such as a liquid, e.g. for oral administration, or an injectable dosage.

An exemplary formulation may comprise PPS in a concentration of about 250 mg/mL; sodium bisulfite in a concentration of up to about 10 mg/mL; EDTA in a concentration of about 0.25 mg/mL; and methyl paraben in a concentration of about 1 mg/mL. The formulation may be stable in a pH range of about 5.8 to about 6.2. The formulation may be formulated in any dosage form, such as a liquid, e.g. for oral administration, or an injectable dosage.

Another exemplary formulation may comprise PPS in a concentration of about 250 mg/mL; sodium bisulfite in a concentration of up to about 10 mg/mL; EDTA in a concentration of about 0.25 mg/mL; and methyl paraben in a concentration of about 1 mg/mL. The formulation may be stable in a pH range of about 7.8 to about 8.2. In some embodiments, the pH of the formulation may be adjusted with 1% w/v sodium hydroxide. The formulation may be formulated in any dosage form, such as a liquid, e.g. for oral administration, or an injectable dosage.

According to various embodiments of the invention, the formulations of PPS described herein, such as pharmaceutical PPS formulations, may be used as an anticoagulant and/or to treat conditions such as arthritis, interstitial cystitis, transmissible spongiform encephalopathy (TSE) (such as BSE) and immunodeficiency virus (such as HIV/AIDS or FIV) in mammals, such as humans, food-producing mammals, and companion mammals (such as feline, canine and equine). The formulations described herein may also be used to treat hematomes, hemorrhoids, frostbites, burns, and multiparameter illnesses such as thrombosis and atherosclerosis.

According to various embodiments of the present invention, the PPS formulations described herein may be administered to mammals, such as to a human, horse, dog, cat, or other mammal, in any suitable manner, e.g., to treat any one or more of the above-identified conditions. For instance, the formulations described herein may comprise topical and systemic formulations for oral, intravenous, intramuscular, intra-articular, or subcutaneous administration. Various other embodiments may be formulated to be administered by way of a transdermal patch, a cream, intravenous solution, eye drops, spray, liposomes or any other method of application and ingestion.

According to some embodiments, liquid (e.g., aqueous) PPS formulations may be administered via injection. In some embodiments, liquid PPS formulations may be administered orally. In some embodiments, liquid formulations may not be terminally sterilized.

The PPS formulations of the present invention can be further processed by known methods to produce a pharmaceutically acceptable composition. In certain instances, this may entail using a pharmaceutically acceptable carrier with any of the formulations described herein, whether that carrier is in a liquid or solid format. For example, the formulation can be further processed so as to be administered in any suitable liquid or powder form, such as by pill, capsule, liquid, liposome, lyophilized composition, hard or soft chewable tablet. The formulation may be administered, e.g., to a mammal, in one or more dosage forms. Dosage forms of the formulation may be administered in an amount effective to treat one or more diseases.

The PPS formulations of the present invention may be formulated for administration to a mammal, e.g., for oral or injectable administration, in any of the dose ranges described below.

A dose of the PPS formulations described herein, e.g., a liquid injectable dose, may comprise PPS in an amount of about 10 mg to about 5 g or about 1 mg/kg to about 5 mg/kg, for example. In some embodiments, a dose of about 3 mg/kg may be administered via injection. In some embodiments, the amount of the dosage form comprises an amount sufficient to inject about 1 mg/kg to about 5 mg/kg of PPS at each injection.

In some embodiments, a dose of the PPS formulations described herein, e.g., a liquid dose for oral administration, may comprise PPS in an amount of about 4 mg/kg to about 20 mg/kg. In some embodiments, a dose of about 10 mg/kg may be administered orally. In some embodiments, the amount of the liquid formulation comprises an amount sufficient to deliver an oral concentration of about 4 mg/kg to about 20 mg/kg of PPS at each administration.

A dose of PPS formulation may further comprise an aminosugar in any of the following amounts: about 2 to about 10 mg/kg, about 10 mg to about 5 g, or about 25 to about 500 mg/mL for injection, or about 15 to about 50 mg/kg for oral administration. In some embodiments, an amount of about 6 mg/kg may be administered via injection. In other embodiments, an amount of about 21 mg/kg may be administered orally.

A dose of PPS formulation may further comprise hyaluronic acid in any of the following amounts: about 0.01 to 5 mg/kg, 0.1 to about 50 mg/mL, or about 0.1 to about 3 g for injection or about 0.1 to 10 mg/kg for oral administration. In some embodiments, an amount of about 0.1 mg/kg may be administered via injection. In other embodiments, an amount of about 0.2 mg/kg may be administered orally.

It should be appreciated that smaller doses may be appropriate for humans and small mammals, while larger doses may be appropriate for larger animals. A dosage amount may be based on the mass of the target subject. For example, a dosage may comprise about 3 mg per kg of body mass of the target subject, such as a human or a horse. It will be appreciated by those of ordinary skill in the art that the dosage for a particular formulation depends in part on the salt of PPS in the formulation. For example, formulations comprising sodium PPS may have a different dosage than formulations comprising calcium PPS. Dosage calculations can be determined by those of skilled in the art by evaluating body weight, surface area, and species differences.

According to various embodiments of the present invention, doses may be administered in a variety of frequencies. Oral doses of the formulations described herein may be administered daily, about once every two or three days, about twice weekly, or weekly. Oral formulations according to present invention may be administered for a total duration of about four to about five weeks, for several months, several years, or permanently.

Injectable doses, such as intramuscular, intraarticular, subcutaneous or intravenous dosages, may be administered about daily, about once every two or three days, about twice weekly, about weekly, about bi-weekly, about monthly, or in other administration frequencies. Such doses may be administered for time periods such as about four weeks to about five weeks, about two months, about six months, or other term of treatment.

Doses described herein may also be administered in pulse therapy, e.g., where doses are administered periodically (e.g., about daily) for a period of time such as 1-3 months, then not administered for a period of time such as 1-3 months, and then administered again periodically (e.g., about daily or at some other appropriate interval) for a time period such as 1-3 months. Other dosage regimens are apparent to one of ordinary skill in the art in light of this specification.

It will be appreciated by those of ordinary skill in the art that a single dose of the formulations described herein, such as a single daily dose, may be administered in parts and/or at different times throughout a single day. For instance, a daily dose may be divided so that half is administered twice per day, e.g., half in the morning and half at night or administered three times in a single day.

In some embodiments of the present disclosure, capillary electrophoresis may be used to analyze samples of PPS-containing substances, such as PPS formulations of the present invention. Such analysis may be used to accomplish, for example, any of the following: detect any polymeric degradation products; determine free sulfate as an additional index of degradation; compare different sample lots before and after sterilization; compare a sample with another PPS-containing formulation or a known published sample, such as Cartrophen^{®}.

In some embodiments of the present disclosure, capillary zone electrophoresis can be used to analyze one or more samples of PPS formulations. Capillary zone electrophoresis may be conducted in a free solution. Separation of components during capillary zone electrophoresis may be based on differences in various components' charge-to-mass ratio. A homogeneous buffer solution may be used. A constant electric field may be applied. The process of capillary zone electrophoresis may depend on pH.

Buffer additives for capillary zone electrophoresis may comprise any suitable buffer and may include any of the following: inorganic salts, organic solvents, urea, sulfonic acids, cationic surfactants, cellulose derivatives, amines, organic acids, and organic polymers.

The analytic capability of CE on a PPS formulation in accordance with various embodiments of the invention allows quantification of sulfate in the presence or absence of a known amount of sulfite; quantification of total pentosan levels; detection and quantification of fragments and/or degradation products; and detection and quantification of oligosaccharides.

The procedures used to identify degradation and beneficial PPS formulations are described more fully in the Figures, which show exemplary capillary electrophoresis systems, results showing degrees of stability and degradation achieved in different circumstances, and formulations identified to have desirable properties.

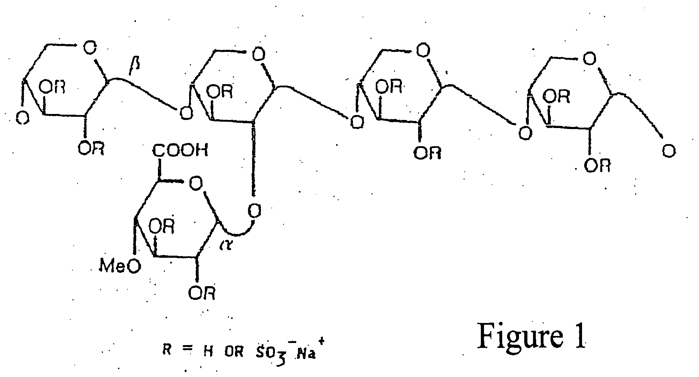

FIG. 1 shows the basic chemical structure of pentosan polysulfate. In the diagram, the R represents either Hydrogen or SO₃⁻Na⁺.

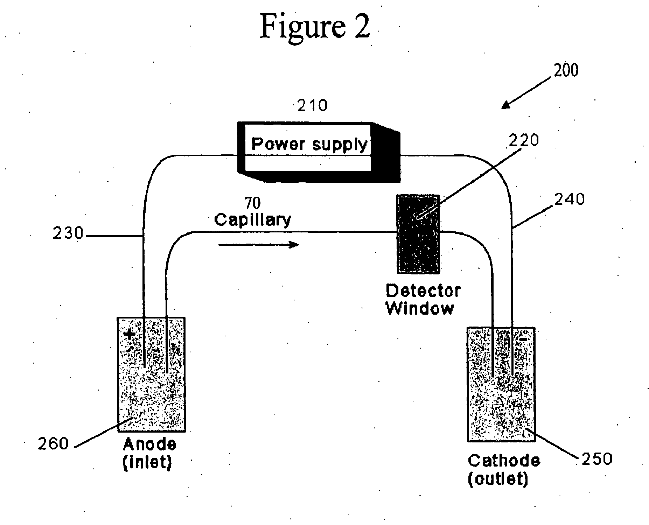

FIG. 2 shows an exemplary schematic of a high-performance capillary electrophoresis (HPCE) instrument 200. The HPCE instrument comprises a power supply 210 having electrodes 230, 240 connected to an anolyte (inlet) 260 and catholyte (outlet) 250. The power supply 210 generates a voltage between the anolyte (+) 260 and the catholyte (-) 250. One end of capillary 70 is immersed along with the anode into the anolyte buffer 260, and the other end is immersed along with the cathode into the catholyte buffer 250. A detector 220 is configured to detect absorption in the capillary 70.

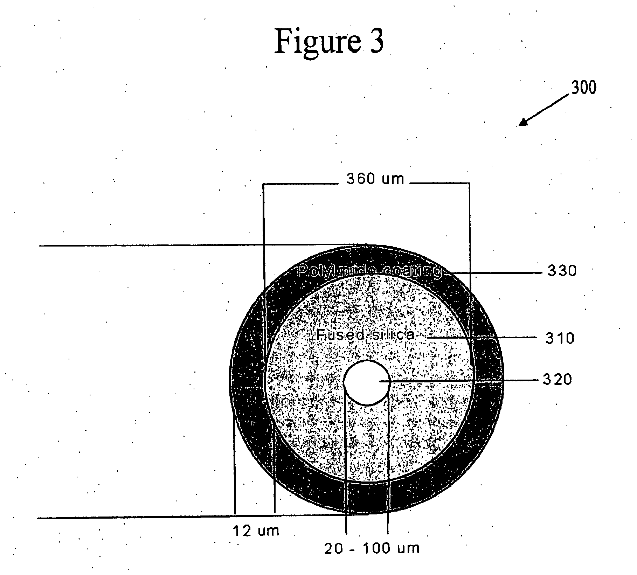

FIG. 3 shows a cross-sectional view of an exemplary capillary 300. The capillary 300 may comprise an inner portion 310 and an outer coating 330. The inner portion 310 may comprise fused-silica, or another suitable material, and it may define a total diameter of approximately 360 µm. The coating 330 may comprise a polyimide coating, or other suitable material, and it may have a thickness of approximately 12 µm. The capillary 300 may be tubular in shape and define a hollow inner shaft 320. The inner shaft 320 may be substantially cylindrical in shape, and it may have a diameter of approximately 20-100 µm.

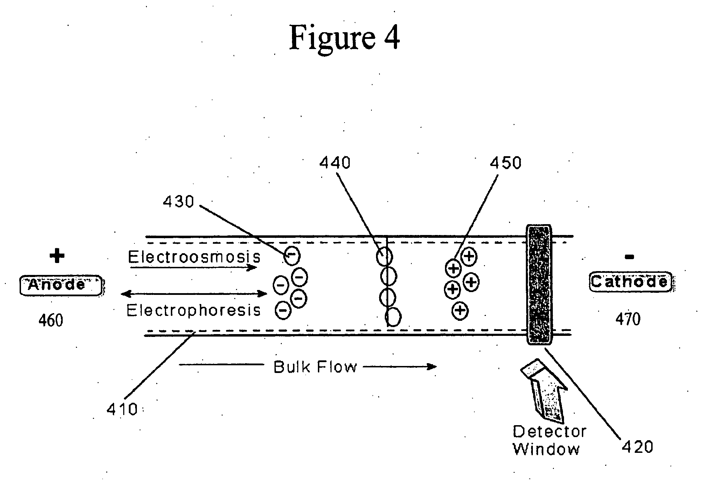

FIG. 4 shows an exemplary schematic of electrophoresis and electroosmosis in a separation of anionic 430, neutral 440, and cationic 450 analytes. After injection of a sample into the tube, the right end of the tube 410 and an electrode (the cathode 470) are placed in a buffer reservoir, while the left end of the tube 410 and another electrode (the anode 460) are simultaneously placed in another buffer reservoir. A voltage is applied between the electrode and cathode. As depicted in FIG. 4, the bulk fluid in the tube 410 flows with the positively charged, hydrated cations in a process called electroosmosis or electroosmotic flow (EOF). Other positively charged analytes move in the same direction. Negatively charged, anionic molecules move in the opposite direction towards the cathode. If EOF exceeds electrophoretic mobility for a group of anions, the anions will move in the other direction. The polarity of the electrodes may be reversed to ensure that the negatively charged anions in the sample pass the detector window 420.

Electrophoretic mobility and EOF effectively cause the different components of the sample to travel through the tube 410 at different rates. Accordingly, different molecule types reach the detector window 420 at different times. When a particular light-absorbing molecule flows past the detector window 420, its passage can be detected directly as a change in light absorption. If the molecule is not light-absorbing (like pentosan) but displaces a similarly charged, light absorbing buffer component, it may be detected indirectly as a decrease in light absorption by the buffer.

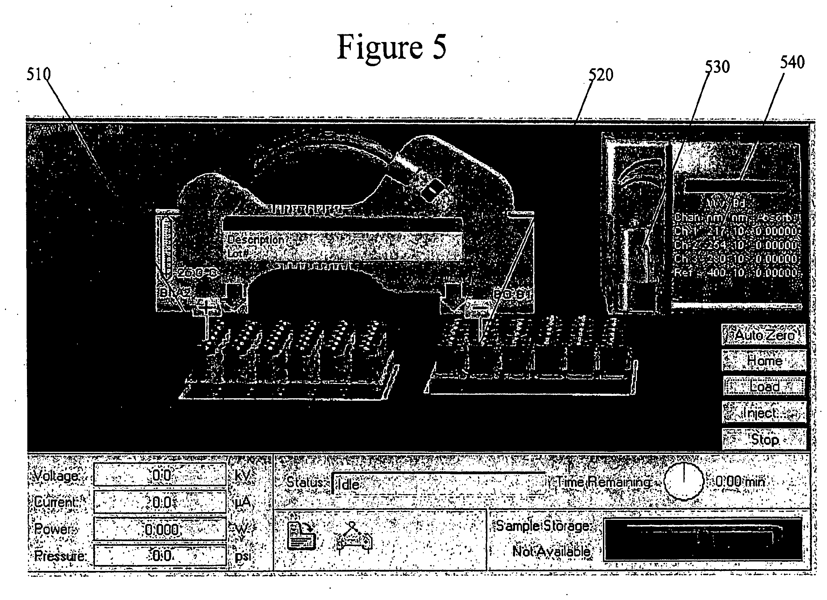

FIG. 5 shows an exemplary system for conducting electrophoresis measurements on sample formulations. More specifically, FIG. 5 shows a printout of the loading screen from the 32 Karat Software package (version 5, build 1021). An exemplary inlet port 510 and outlet port 520 are depicted, along with the deuterium lamp 530 and wavelengths 540 of light output by the deuterium lamp 530.

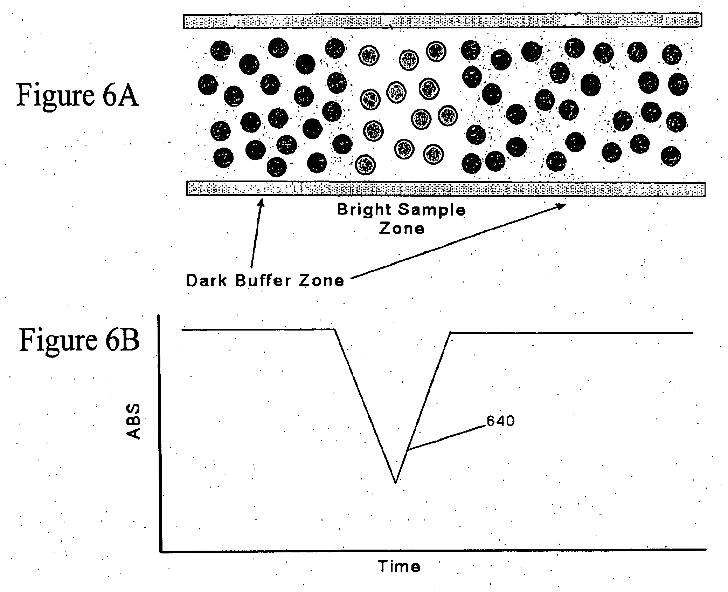

FIG. 6A shows that as the sample and buffer move through the capillary, the similarly charged, non-light-absorbing sample displaces the light-absorbing buffer, creating a zone of reduced buffer concentration. As shown in the graph of FIG. 6B, as the sample passes the detector window, the detector will detect a reduction in light absorption 640 followed by a return to a baseline light absorption after the sample passes. Because different molecules effectively travel through the tube at different rates and reach the detector window at different times, they can be separated and distinguished by detecting light absorption at the detector window 420.

It should be appreciated that electropherograms obtained using indirect detection methods may be inverted for convenience in viewing results. For instance, PPS may trigger a "valley" that is inverted to form a peak. Regardless of the graphical representation, the PPS "valley" or "peak" represents a substantial deviation from the "baseline". Recognizing this interchangeability, CE representations of PPS will be referred to as "peaks" rather than "valleys" for purposes of this specification.

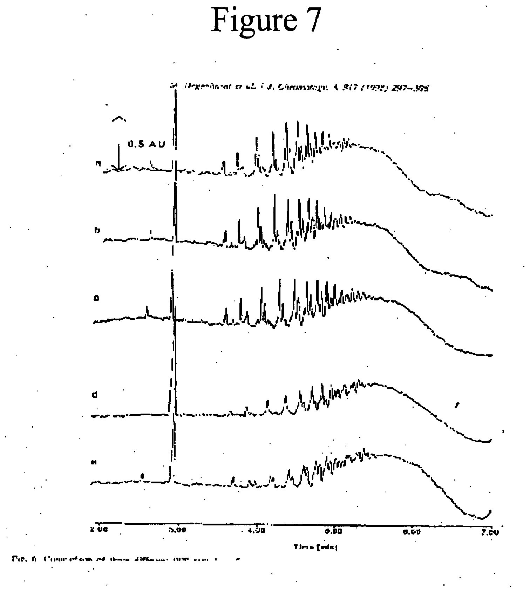

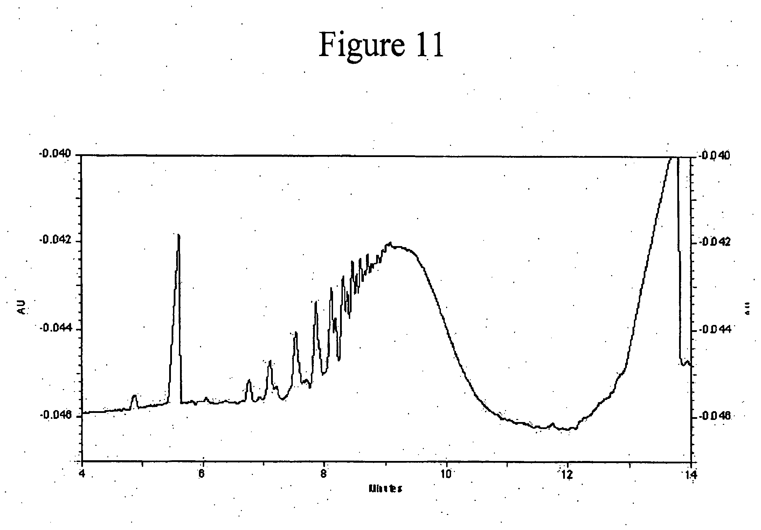

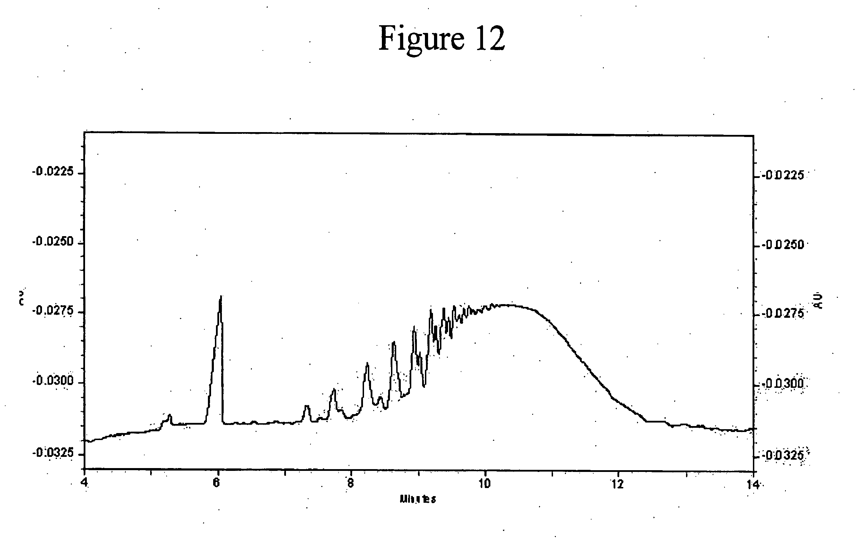

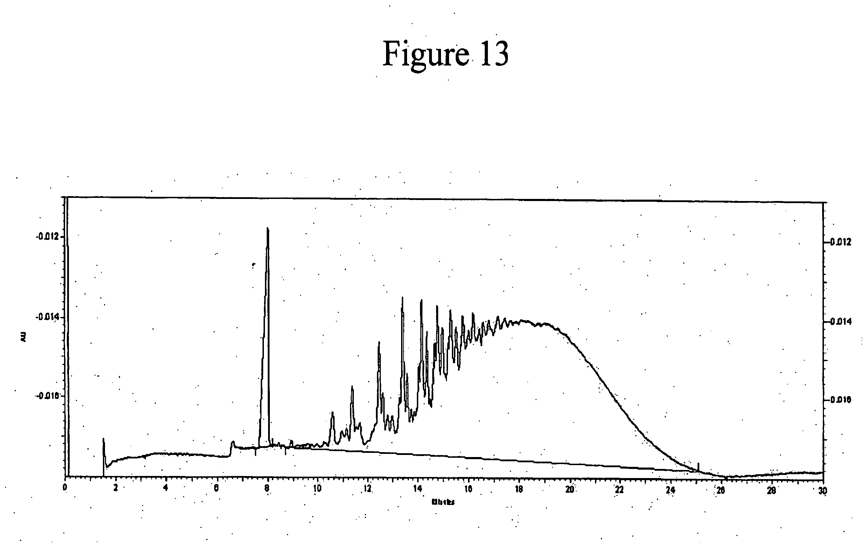

Generally speaking, the electropherograms shown in FIGS. 7-13 are characterized by a generally bell-shaped primary absorption peak over a time period of several minutes and a plurality of other absorption peaks over much smaller periods of time. The primary peak generally appears near the middle of these electropherograms. Notably, in many of the electropherograms the primary bell-shaped absorption peak has a plurality of successive absorption peaks on its leading edge, the leading edge being the left part of the electropherogram curve that is detected earlier in time. These peaks likely correspond to smaller molecular weight molecules of PPS. Because they are smaller, they can travel more quickly through the capillary and are therefore detected earlier in the electropherogram than the primary peak for PPS, corresponding to the top of the bell-shape.

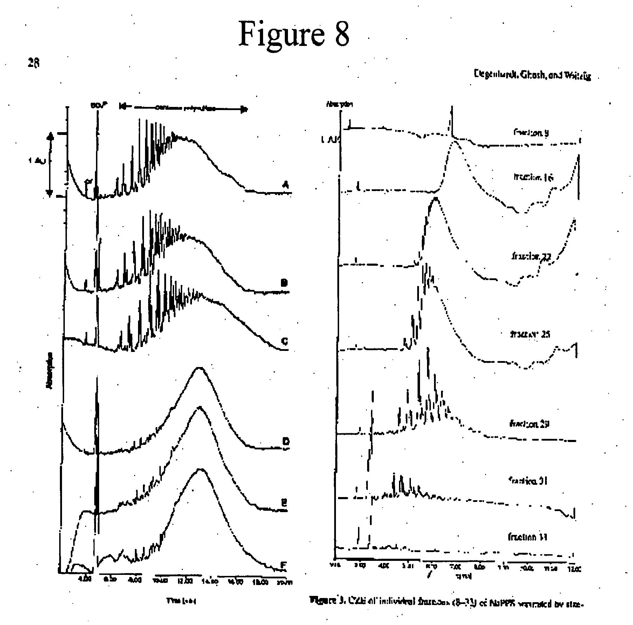

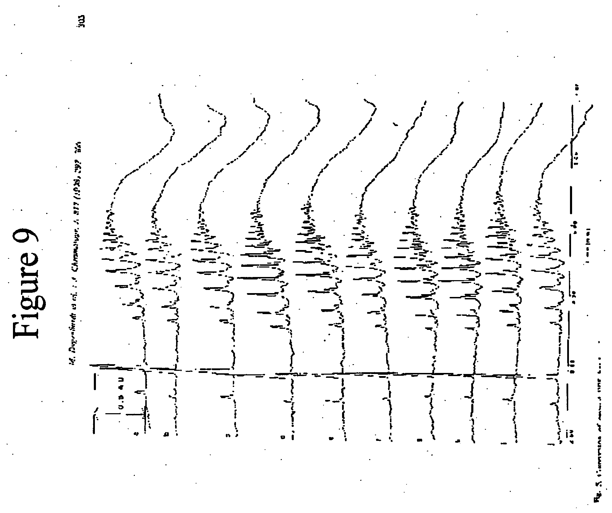

FIGS. 7-9 show electropherograms for PPS-containing substances that were not formulated according to the present invention. These electropherograms are change-in-absorption-versus-time curves generated using capillary zone electrophoresis (CZE). FIGS. 7 and 9 are copied from Degenhardt, Benend et al. (1998). FIG. 7 shows capillary electrophoresis analysis of Cartrophen^{®} and an unidentified PPS product. FIG. 8 is copied from Degenhardt, Ghosh et al. (2001), and it shows curves generated using CZE. Curves D, E, and F on the bottom left of FIG. 8 are relatively smooth bell-shaped curves that show CE analysis of PPS samples having substantially homogeneous molecular weights. The PPS portion of these curves (corresponding to the general bell-shape near the center of each graph) are substantially free from distinct peaks in the PPS portion of the graph (other than the primary PPS peak at the top of the bell).

Accordingly, the presence of these peaks on the leading edge of the wide PPS peak in various electropherograms of FIGS. 7-9 indicates oligosaccharides having different (or heterogeneous) molecular weights. Each successive peak in the left portion of the PPS bell-shaped curve indicates a different molecular weight (or different set of molecular weights). It has been argued that this property of heterogeneity (corresponding to the multiple peaks on the left side of the bell-shape) is necessary for biological effectiveness. However, it is believed that various embodiments of the present invention, having PPS of substantially homogeneous molecular weights, is also biologically effective. Accordingly, the present invention provides PPS formulations that have reduced levels of heterogeneous oligosaccharides therein, as evidenced by fewer peaks on its leading edge. For these formulations, the PPS molecules are said to have substantially similar (or substantially homogeneous) molecular weights.

An exemplary graph showing impurities of a PPS sample that was not formulated according to the present invention is the top-most graph in FIG. 7. This graph shows a series of peaks over short time periods that gradually transition into a more smooth curve over a significantly longer time period. The smooth curve is generally bell-shaped, although the leading (left) side of the "bell" exhibits a series of peaks. In FIG. 7, the peaks actually reach higher absorption levels than the dominant bell-shaped curve corresponding to larger PPS molecules. This indicates an increased presence of heterogeneous oligosaccharides in the sample. In the bottom-most graph of FIG. 7, the peaks are much smaller in relation to the dominant PPS curve, thereby indicating a smaller percentage of heterogeneous oligosaccharides.

FIG. 8 shows six absorption curves for PPS samples that were not formulated in accordance with the present invention, the top three corresponding to one manufacturer and the bottom three corresponding to another manufacturer. As shown by the legend at the top of the graph, the PPS portion of the curve corresponds to the absorption from approximately 7 minutes to approximately 17 minutes. The three top-most curves indicate a significant presence of small oligosaccharides. These top-most curves show sizable leading edge peaks, thereby indicating a relatively heterogeneous composition, i.e., wider range of molecular weights. The bottom three curves indicate substantially no small oligosaccharides (or peaks) and a more homogeneous structure, i.e., PPS molecules having a more symmetric bell-shaped distribution of molecular weights. Accordingly, the smoother bottom-most curves indicate the presence of PPS molecules having a substantially homogeneous molecular weight, i.e., the absence of oligosaccharides having heterogeneous molecular weights.

In comparing the various electropherograms, it should be appreciated that the portion of the graph corresponding to PPS is often bell-shaped and wider than any region of the graph corresponding to a single peak. Although the peaks often distort the overall "bell" shape present in many of these graphs, a general bell-shape can still be determined, as it is characterized by an overall increase in the y-axis, a peak (corresponding to PPS rather than any single peak), and then an overall decrease in the y-axis over a time period that is substantially larger than that for any single peak. Accordingly, the bell-shape corresponding to PPS and its peak near the center of the "bell" should not be confused with any particular peak or peaks that may be present in the graph.

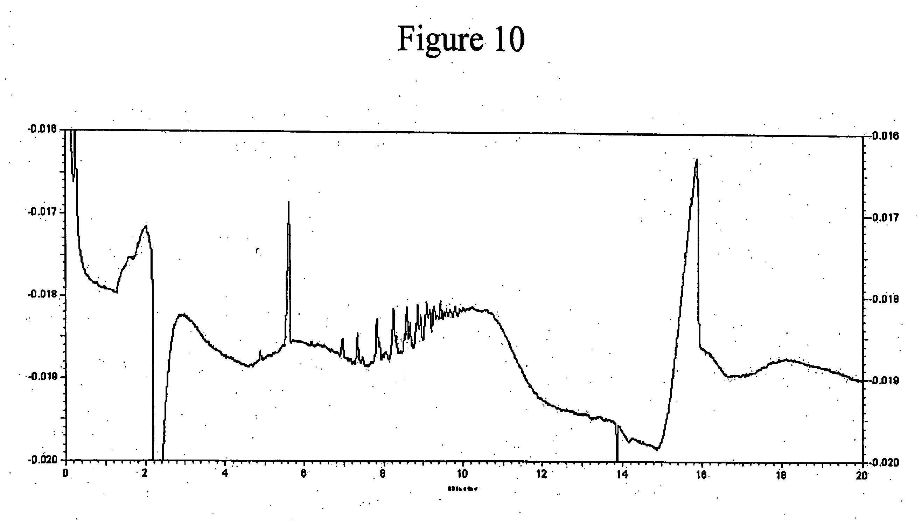

FIGS. 10-13 show several CE absorption curves for a single sample of Cartrophen^{®}, a commercially available PPS-containing substance.

For the CE analyses described herein, a Beckman-Coulter Pace/MDQ capillary electrophoresis system was used to analyze the formulations comprising PPS. The method used was substantially similar to the method outlined by Degenhardt et al. in "Quality control of pentosane polysulfate by capillary zone electrophoresis using indirect detection," Journal of Chromatography A 817 (1998). Fused silica columns were used. The capillary was pretreated with 1 M NaOH. Electrophoresis was accomplished using benzene-1,2,4-tricarboxylic acid (BTC). The BTC was prepared using 368 mg BTC (obtained from Sigma) and 50 mL deionized water. The pH was adjusted to 4.9 using 0.1 M NaOH. Water was added to 200 mL, resulting in a final concentration of 8.75 mM BTC buffer at pH 4.9. Electrophoresis was accomplished using benzene-1,2,4-tricarboxylic acid (BTC). The BTC was prepared using 368 mg BTC (obtained from Sigma) and 50 mL deionized water. The pH was adjusted to 4.9 using 0.1 M NaOH. Water was added to 200 mL, resulting in a final concentration of 8.75 mM BTC buffer at pH 4.9.

The capillary was rinsed with running buffer for 60 min before the first capillary electrophoresis measurement, and then 10 minutes between CE measurements with running buffer. All reagents and samples were filtered through a 0.45 micron filter prior to use. Injection was accomplished for 20 sec using 0.5 psi pressure. The capillary was dipped in BTC to rinse away any sample residue on the outside of the capillary. CE was run at 20 kV for 20 to 40 minutes.

Reversed polarity was applied as described in Degenhardt (1998). Absorption of the background electrolyte was monitored at a 217 nm wavelength. The effectiveness of molecular weight separation in the capillary was determined by analyzing the separation of the different oligosaccharides of Cartrophen^{®}. The Cartrophen^{®} samples were diluted with water from 100 mg/mL to 3 mg/mL. The capillary had the following properties: detection 217 nm, 67 cm total capillary length, 50 cm effective length, 50 µm inside diameter.

FIGS. 10-13 represent the results of applying these methods to a solution containing Cartrophen^{®} in a concentration of about 3 mg/mL of water. It should be appreciated that similar results may be obtained using PPS sample concentrations of about 1 to about 5 mg/mL. These figures depict progressively more resolved electropherograms of a single sample, obtained by extending the migration times with additional column conditioning. Like many of the figures discussed above, the leading edge peaks on the PPS peaks of these graphs indicate the presence of small oligosaccharides.

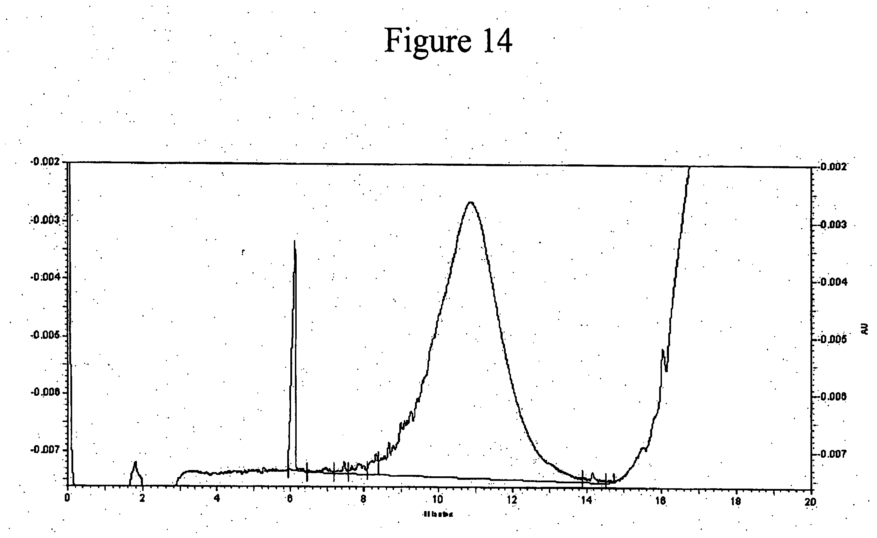

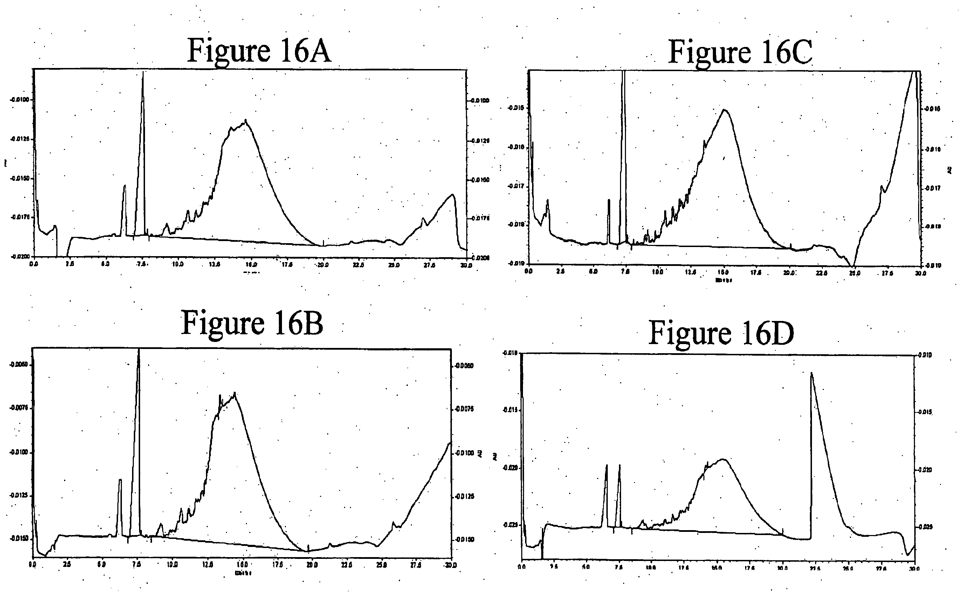

FIG. 14 shows an exemplary electropherogram of PPS raw material alone, present in water in a concentration of about 2 mg/mL. The PPS was obtained from Nature Vet, Australia (lot number M62004037). The relatively smooth bell-shaped curve in the electropherogram shows a lack of small oligosaccharides and a substantially homogeneous molecular weight of the PPS. In this graph it is clear that the height and area of any peaks on the leading edge of the bell-shape of the curve are insignificant compared to the size and area of the overall bell-shaped curve corresponding to PPS. This same phenomenon can be observed in other electropherograms corresponding to various embodiments of the invention as described below, e.g., in FIGS. 16A-17B.

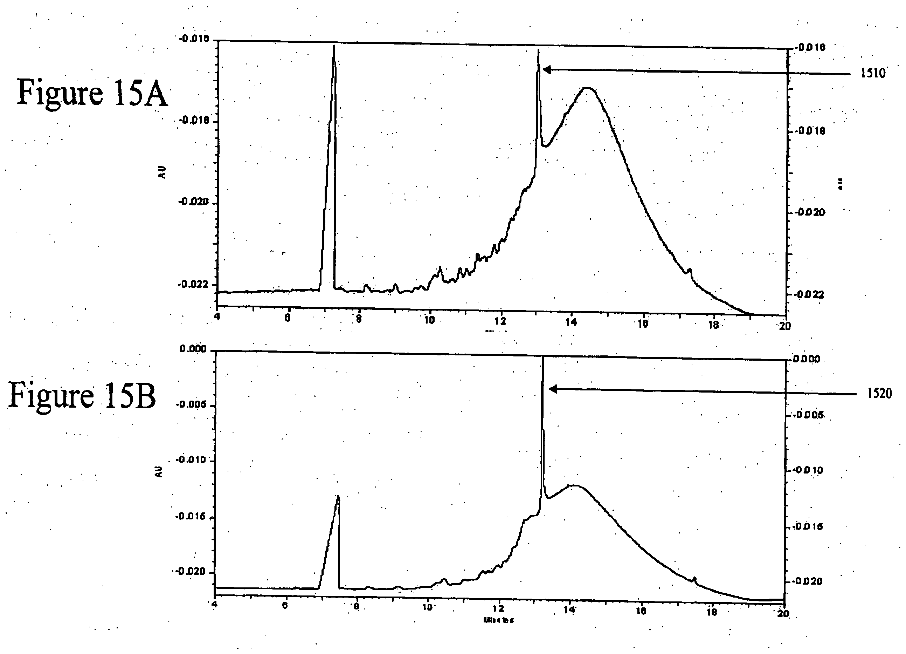

FIGS. 15A and 15B show electropherograms for a commercially available PPS formulation more than two years after the formulation was prepared. Prior to CE analysis, this formulation was aged for over two years after it was created. During a substantial portion of that time period, the sample was not refrigerated and was exposed to temperatures significantly higher than room temperature (i.e., higher than 18-27 degrees Celsius). Over that time period, the formulation's color turned to dark brown from an original color of straw to light yellow. The two curves in FIGS. 15A and 15B look slightly different due to scaling of ordinates to show all of the peak at approximately 15 minutes. The content of this formulation (lot number G103 from Nature Vet, Australia) is described in Table 1, below. In some embodiments, this formulation may be used for equine treatment, e.g., via injection.

**Table 1**

| **Components** | **Amount (per mL)** |
|---|---|
| Sodium Pentosan Polysulfate | 250 mg |
| Potassium Phosphate Monobasic (buffer) | 6.8 mg |
| EDTA (chelating agent) | 0.25 mg |
| Sodium Hydroxide (pH adjuster) | to pH 6.2 - 6.5 |
| Benzvl Alcohol (preservative) | 0.01 mL |
| Water for Injection (diluent) | q.s. ad 1.0 mL |
| Sterility | Sterile |
| LAL | < 0.2EU |
| Glass Vial 6mL (clear) | To EP Standard |
| Halo Butyl Isoprene Stopper 13 mm | To EP Standard |
| Flip-off cap | to commercial standard |

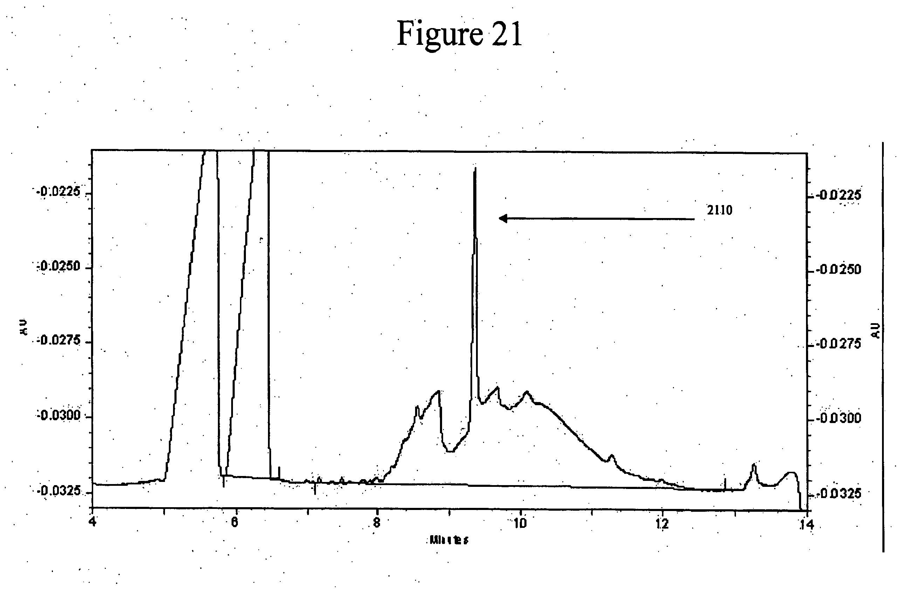

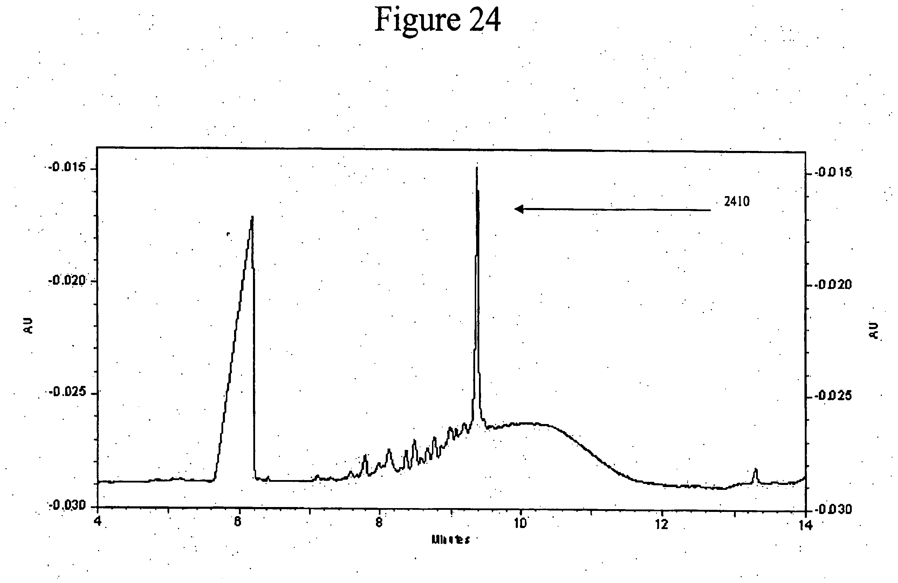

Each electropherogram in FIGS. 15A and 15B exhibits a single high, sharp peak 1510, 1520 near the middle of the PPS peak, indicating a suspected degradation product. These peaks are sometimes referred to as "degradation peaks." Here, each of the peaks 1510, 1520 in FIGS. 15A and 15B is on the left-middle part of the PPS peak. A similar single high, sharp peak is also present in FIGS. 21 and 24, which correspond to PPS-containing formulations of the present invention, and FIG. 24, which corresponds to a sample of Cartrophen^{®}, each subjected to forced degradation by treatment with sodium hydroxide. Accordingly, these degradation peaks in FIGS. 15A and 15B show an example of the degradation that can occur in formulations of PPS over time, e.g., for a commercially available formulation.

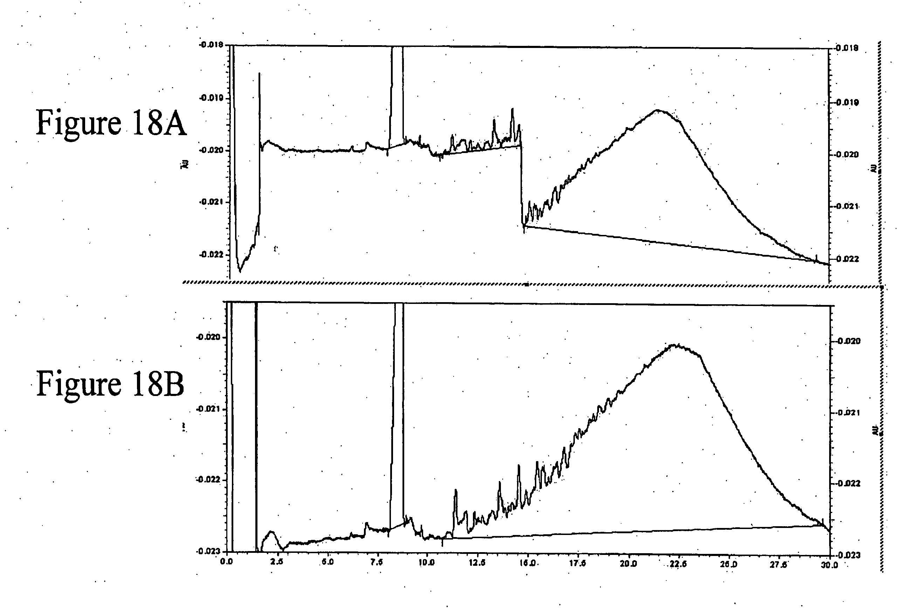

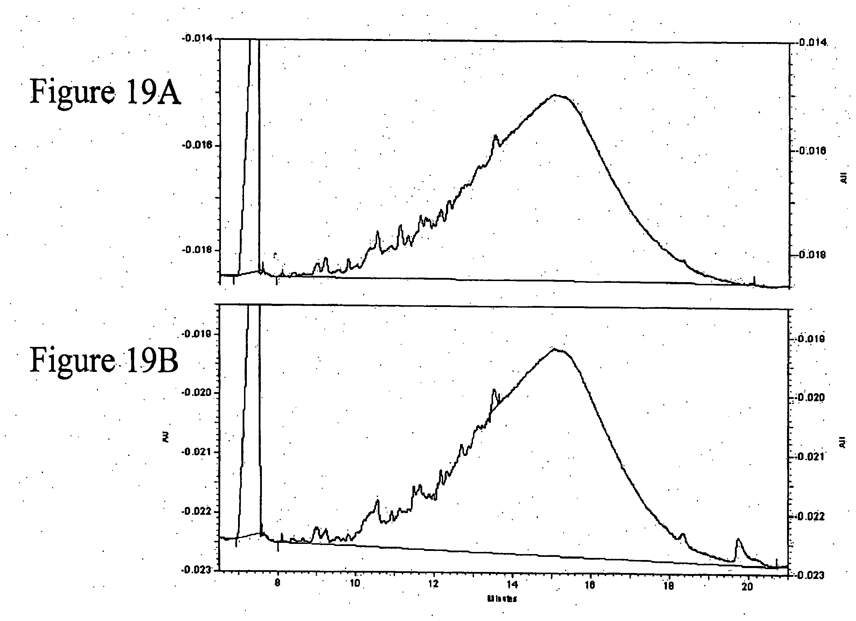

FIGS. 16A-20B show capillary electrophoresis analyses of twelve exemplary formulations of PPS. The twelve formulations are identified below in Table 2. Samples 1B, 1C, 2B, 2C, 3B, 3C are embodiments of the invention, samples 1A, 1D, 2A, 2D, 3A, 3D are not part of the claimed invention.

These formulations were prepared in accordance with the methods described above

Those of ordinary skill in the art will appreciate that some or all of the sodium metabisulfite converts to sulfur dioxide and sodium bisulfite upon addition to the formulation. It should also be noted that the third column from the left, representing pH, is an expected pH rather than a measured pH. The measured pH ("mpH") is provided in the last column on the right. Although no pH was actually measured for the formulations of samples 2 and 3, these samples should be nearly identical to those measured for the formulations of group 1 due to their chemical similarity. Thus, the pH for formulations 2B and 3B are expected to be substantially the same as that measured for 1 B, i.e., approximately pH 4.15.

**Table 2**

| Sample | Group | pH | Composition (mg/mL) | | | | | | |
|---|---|---|---|---|---|---|---|---|---|
| | | | Pentosan | Sodium Metabisulfite | EDTA | NaCitrate | Citric Acid | Methyl Paraben | mpH |
| | | | 250 | 10 | 0.25 | 14.7 | 10.5 | | |
| 1 | A | 4 | X | X | | | | | 3.15 |
| | B | 6 | X | X | | | | | 4.15 |
| | C | 8 | X | X | | | | | 7.46 |
| | D | 4 | X | | | X | X | | 3.73 |
| | | | | | | | | | |
| 2 | A | 4 | X | X | X | | | | |
| | B | 6 | X | X | X | | | | |
| | c | 8 | X | X | X | | | | |
| | D | 4 | X | | X | X | X | | |
| | | | | | | | | | |
| 3 | A | 4 | X | X | X | | | X | |
| | B | 6 | X | X | X | | | X | |
| | c | 8 | X | X | X | | | X | |
| | D | 4 | X | | X | X | X | X | |

Accordingly, twelve formulations were evaluated, including four from sample 1 (1A-1D), four from sample 2 (2A-2D), and four from sample 3 (3A-3D). These twelve formulations appear to comprise a more homogenous blend of molecular weights rather than a more polydisperse mixture of a wider range of molecular weights as was found in other formulations such as Cartrophen^{®}, which has been shown to contain a mixture of PPS molecules with a range of sizes that appear to include discrete, smaller oligosaccharides along with larger sizes. Of these twelve, formulations 1A-1C, 2A-2C, and 3A-3C were identified to have especially desirable properties. In particular, these nine formulations also did not show degradation or a color change after terminal sterilization or storage (e.g., storage without refrigeration, such as at room temperature). Finally, formulations 1B, 1C, 2B, 2C, 3B, and 3C have a more physiologically compatible pH range that is expected to be non-irritating when injected.

It should be noted that the three "A" groups were measured to have substantially similar properties under CE analysis across all samples, as did the "B," "C," and "D" formulations. In other words, the electropherograms were substantially identical for 1C, 2C, and 3C, for example.

The graphs in FIGS. 16A-20B show substantially smooth bell-shaped curves, indicating that the PPS in these formulations are substantially free from oligosaccharides having heterogeneous molecular weights or impurities such as smaller oligosaccharides. As noted earlier, the results for each group (A-D) were consistent across all samples (1-3). In other words, the results for formulation 1B were substantially identical to the results obtained for formulations 2B and 3B.

Physical observation of the samples through these tests revealed that all the samples in groups "A," "B." and "C" remained substantially clear and free from discoloration. Prior art samples, as well as samples in group "D," have been observed to turn brown under similar conditions. At present it is not known exactly what causes discoloration such as the observed brown discoloration, although it clearly represents a change in the chemical formulation of the sample and is therefore an indication of degradation.

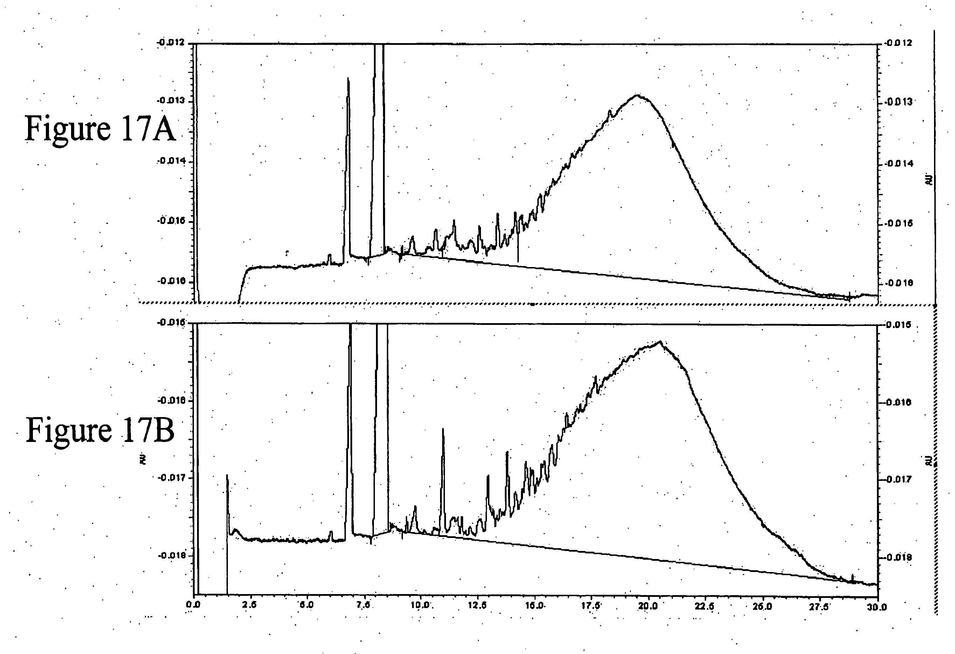

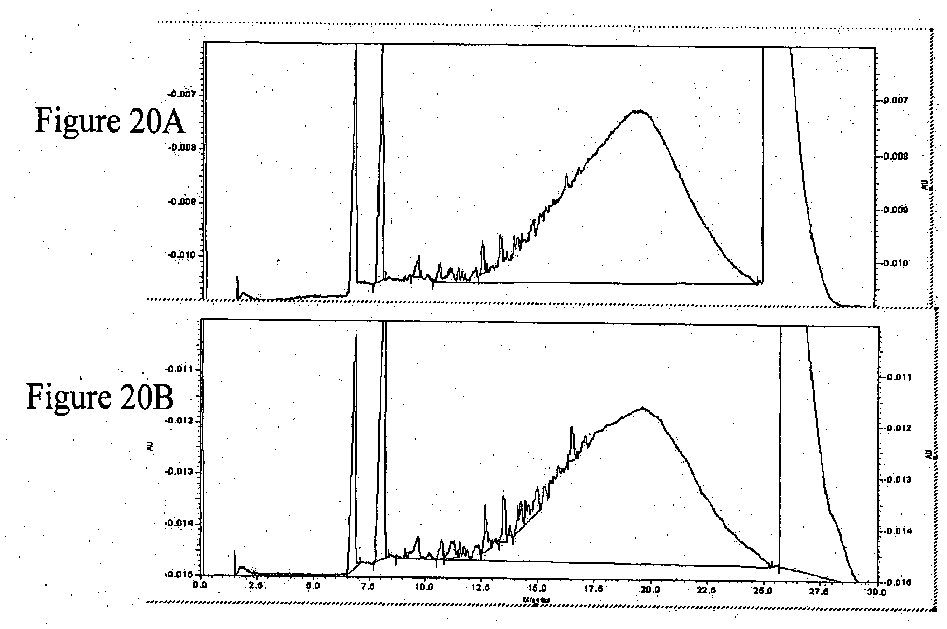

FIGS. 17A-20B show electropherograms for samples A-D, respectively, before and after terminal sterilization. The figures labeled with an "A" (as in FIGS. 17A, 18A, 19A, and 20A) show electropherograms for samples that were not sterilized prior to measurement. The figures labeled with a "B" (as in FIGS. 17B, 18B, 19B, and 20B) show electropherograms for samples that were terminally sterilized prior to measurement. Thus, for example, FIGS. 20A and 20B show electropherograms for formulation "D" before and after sterilization, respectively.

To obtain these results, CE measurements were taken for two vials of each of the 12 mixtures (shown in Table 2). One vial was non-sterile, and the other was terminally sterilized at 121°C. for 15 minutes. The PPS concentration was initially 250 mg/mL for each vial. The group A samples were observed to have a pale straw color. The group B samples were observed to have a straw color. The group C samples were observed to have a yellow color. All samples were diluted to 2.5 to 3 mg/mL in water in 200-250 µL of sample in injection vials. CE analysis was conducted by applying 20 kV for up to 40 min.

The electropherograms for the sterilized and un-sterilized samples show that the PPS peak was substantially undisturbed by sterilization. In the electropherograms for each sample, the PPS portion of the curve (i.e., the general bell-shaped curve over several minutes having a gradual peak in the middle of the "bell") does not show appreciable degradation products either before or after sterilization. (It should be noted that, in order to verify that an electropherogram shows a substantially smooth bell-shaped curve, it is recommended to first verify that the CE methods used to obtain the results are capable of detecting heterogeneity in other samples.)

In addition, the group A, B, and C samples did not discolor after sterilization. However, the group D sample was observed to turn dark after sterilization. Also, as shown in its electropherogram in FIG. 20B, the group D sample exhibited an increase in peaks on the leading edge of the PPS curve after autoclaving, although the peaks are still relatively small in comparison to the bell-shape of the PPS curve. A large peak near 22-25 minutes appears in all group D sample electropherograms. A similar-looking peak occurred near 22-25 minutes in a sodium citrate solution pH adjusted to 4.0 with HC1 and to 14.7 mg/mL, diluted to 147 µg/mL to match the group D samples. Thus, the similar-looking peak in the group D sample electropherograms at 22-25 minutes likely corresponds to the citrate which is present in the group D samples but not the other samples.

According to various embodiments of the invention, CE analysis may be used to detect degradation products in PPS formulations, e.g., after forced degradation of the sample. For example, various formulations of the present invention were subjected to forced degradation. CE analysis of these samples showed the presence of a degradation peak, thereby indicating degradation of the PPS as a result of forced degradation. CE analysis was also used to show that at least one commercially available formulation exhibits degradation after it is subjected to degradation conditions such as aging and heat (see FIGS. 15A and 15B).

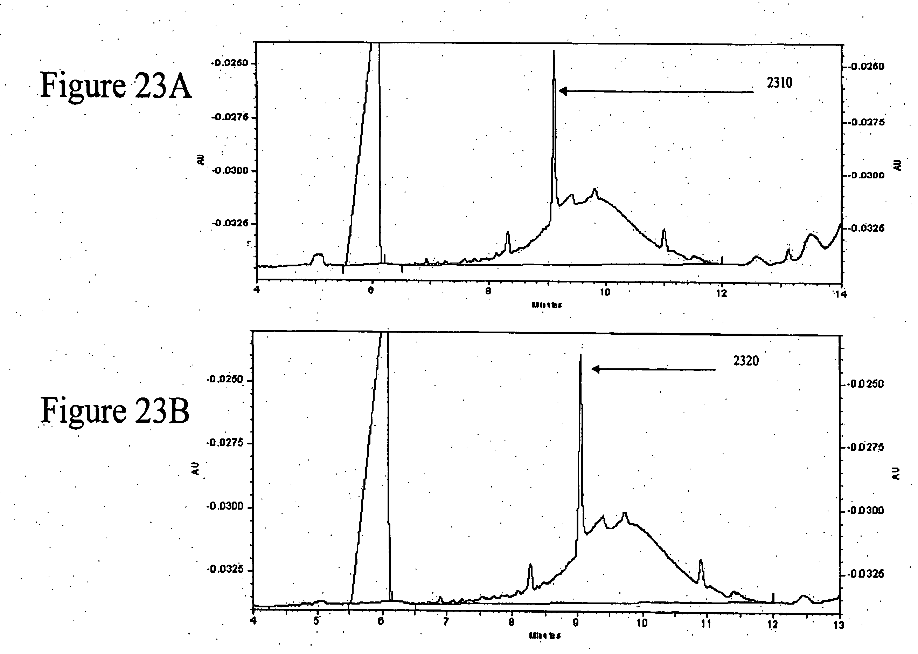

FIGS. 21, and 22A-23B show electropherograms of group C samples after forced degradation. FIGS. 21, 23A, and 23 B each show the presence of a tall peak 2110, 2310, 2320 occurring between 9 and 10 minutes that indicates a degradation product. A similar degradation peak 1510 is observed in the naturally degraded PPS seen in FIGS. 15A and 15B. It was additionally observed that the group "C" samples remained substantially clear and did not exhibit brownish or other discoloration throughout these forced degradation tests. Prior art PPS formulations have been observed to turn brown over time with the presence of a degradation product visible on the electropherogram (e.g., FIGS. 15A and 15B).

FIG. 21 shows an electropherogram for a group "C" sample after forced degradation with 0.1 N sodium hydroxide for 24 hours at 60°C., neutralized with HCl and sodium acetate. (A vial containing a group C sample was placed in a hot water bath maintained at 60°C. for 24 hours prior to CE analysis.) The graph shows a substantially bell-shaped curve indicating the presence of PPS. The first large peak in the graph represents the chloride anion from the hydrochloric acid used for neutralization before testing. The second large peak represents sulfate. The amount of sulfate (as indicated by sulfate peak height in the electropherogram) is increased due to free sulfate generated during forced degradation. The electropherogram shows additional peaks at 8-9 min which are associated with chloride and are not derived from pentosan. (It is believed that these peaks represent a chloride-associated anion because they appear when NaCl is tested.) The remaining curve is essentially bell-shaped except for the sharp, discrete peak in the middle of the pentosan portion of the graph, indicating a degradation product.

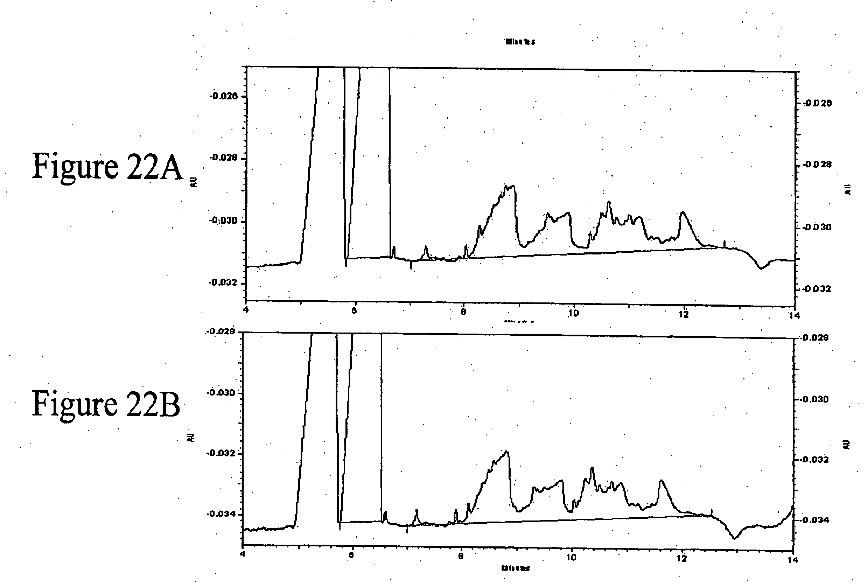

FIGS. 22A and 22B show electropherograms of samples 1C (FIG. 22A) and 2C (FIG. 22B) after 24 hours of exposure to 0.1 N HCl acid and heat (60°C.). Degradation under acidic conditions is more severe than basic conditions and alters the electropherogram, such that it no longer maintains a homogenous distribution of PPS molecules.

FIGS. 23A and 23B show electropherograms of samples 1C and 2C, respectively, after forced degradation with 0.1 N sodium hydroxide for 24 hours at 60°C., neutralized with acetic acid. As in FIG. 21, the electropherograms of FIGS. 23A and 23B show a substantially bell-shaped curve indicating the presence of PPS. A sharp peak from a degradation product is also present.

FIG. 24 shows an electropherogram of a Cartrophen^{®} sample after forced degradation with 0.1 N sodium hydroxide for 24 hours at 60°C. In comparison to the corresponding forced degradation of the group "C" samples, there is substantially less PPS present, as indicated by the smaller bell-shaped PPS curve and corresponding shorter peak. As in FIGS. 23A and 23B for samples 1C and 2C, the electropherogram in FIG. 24 shows a degradation peak 2410 in Cartrophen^{®} after forced degradation. Furthermore, the Cartrophen^{®} sample was observed to discolor by turning a light brown color.

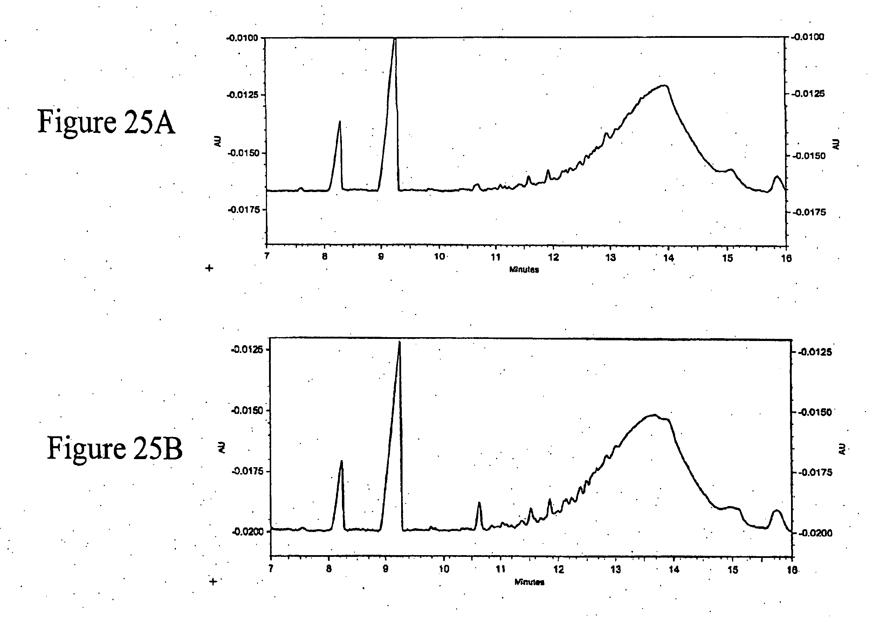

FIGS. 25A-28B show electropherograms for sterilized and un-sterilized samples comprising PPS that were stored at different temperatures. In these and later analyses the effective capillary length was increased from 50 cm to 70 cm, resulting in longer migrations for sulfate and PPS components.

FIGS. 25A and 25B show electropherograms of formulation 1A (Table 2) un-sterilized (FIG. 25A) and sterilized by autoclaving for 15 minutes at 121°C. (FIG. 25B), then stored at 5° for 3 months. As shown in FIGS. 25A and 25B, the electropherograms for the sterilized and un-sterilized samples are very similar. There appears to be little effect of sterilization on the formulation of the present invention, with a slight increase in the sulfate peak and slight increase in the oligosaccharide peak at 10.5 minutes. Neither sample changed colors or otherwise exhibited the light brown discoloration that characterized the Cartrophen^{®} sample after forced degradation.

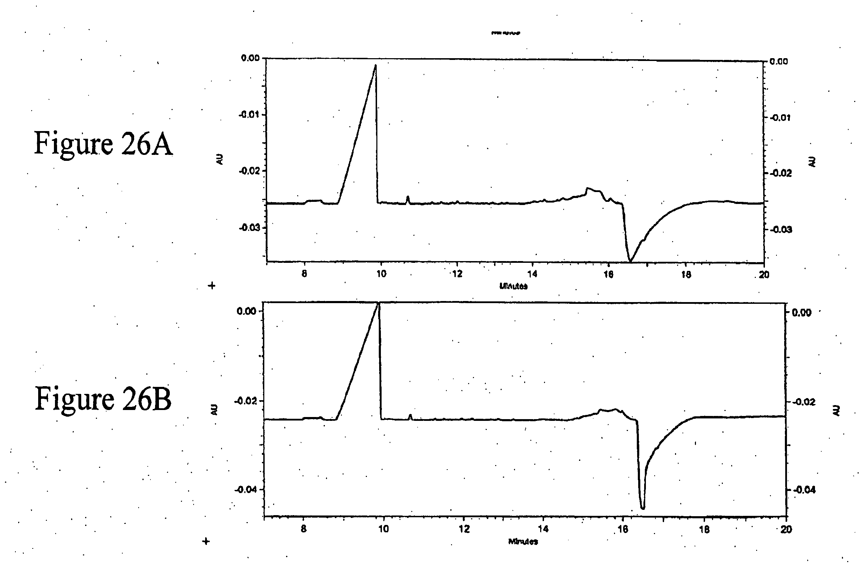

FIGS. 26A and 26B show electropherograms of formulation 1A (Table 2) un-sterilized (FIG. 26A) and sterilized by autoclaving for 15 minutes at 121° C. (FIG. 26B), then stored at 40° C. and 75% relative humidity for 3 months. The electropherograms of FIGS. 26A and 26B show almost no indication of a pentosan peak, but they do show a very large increase in the sulfate peak. Here, applying the CE method to these samples shows degradation of the PPS in the samples in a high temperature environment over time.

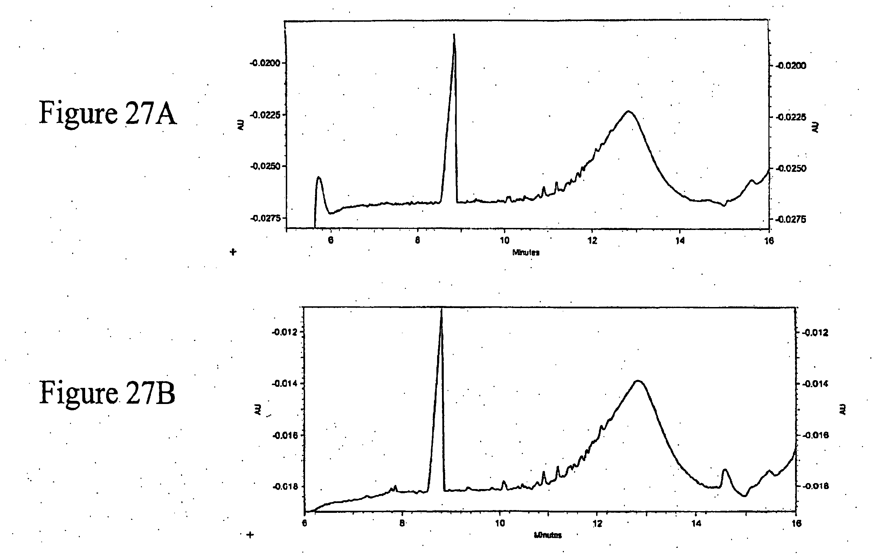

FIGS. 27A and 27B show electropherograms of formulation 1B (Table 2) un-sterilized (FIG. 27A) and sterilized by autoclaving for 15 minutes at 121° C. (FIG. 27B), wherein both formulations were then stored at 5° C. for 3 months. As shown in FIGS. 27A and 27B, the electropherograms for the sterilized and un-sterilized samples are very similar. Sterilization was observed to have little or no effect on the pentosan formulation. The sterilized sample remained stable without any detectable degradation peaks under cold storage conditions. Neither sample changed colors or otherwise exhibited the light brown discoloration that characterized the Cartrophen^{®} sample after forced degradation.

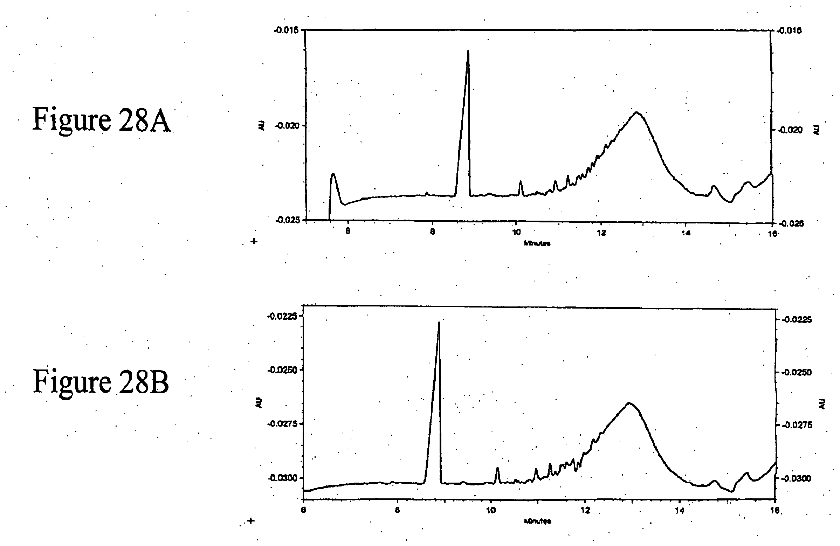

FIGS. 28A and 28B show electropherograms of formulation 1B (Table 2) un-sterilized (FIG. 28A) and sterilized by autoclaving for 15 minutes at 121° C. (FIG. 28B), then stored at 40° C. and 75% relative humidity for 3 months. As shown in FIGS. 28A and 28B, the electropherograms for the sterilized and un-sterilized samples are very similar. Sterilization was observed to have little or no effect on the pentosan formulation. The sterilized sample remained stable without any detectable degradation peaks under high temperature storage conditions. Neither sample changed colors or otherwise exhibited the light brown discoloration that characterized the Cartrophen^{®} sample after forced degradation.

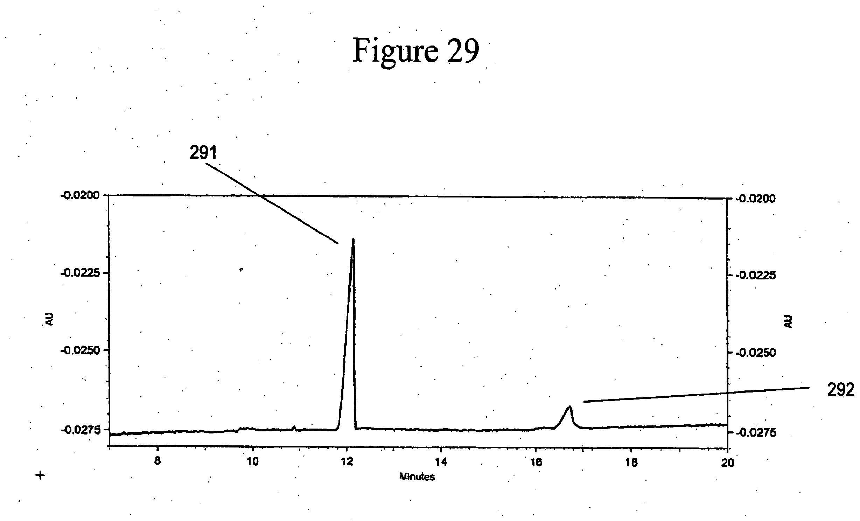

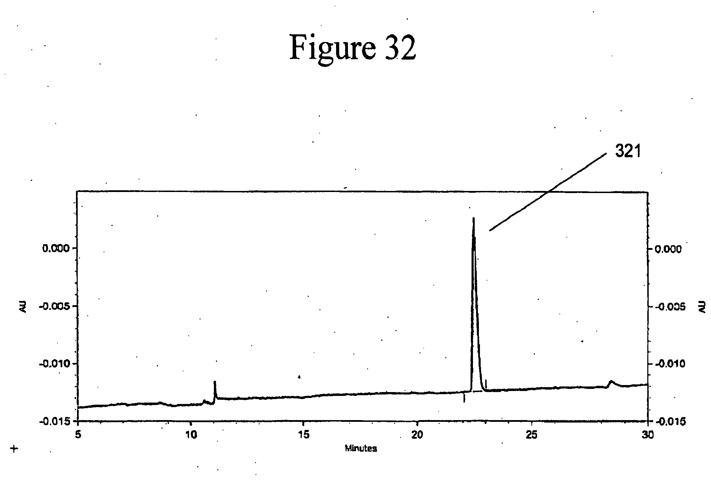

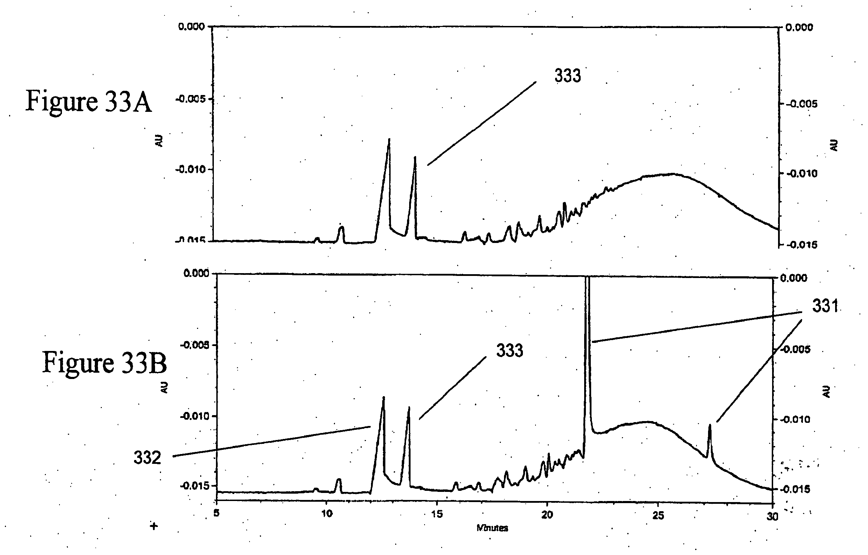

In some experiments, additional steps were taken to further ensure reliable CE measurements and resolution of peaks in a manner sufficient to ensure detection of degradation fragments and/or small oligosaccharides in formulations of pentosan according to the present invention. For instance, for the CE methods used to produce the electropherograms of FIGS. 29, and 30A-33B, each new capillary was conditioned with 1 N NaOH for a period of time, in some cases up to 3 hours. For some samples, an internal standard (see FIGS. 29, 30B and 31B) and/or reference standard (see FIGS. 32 and 33B) was included to ensure capillary quantitation and resolution sufficient for pentosan detection. In addition, it was found that a 50 cm effective length, 50 µm inside diameter capillary shows the sulfate peak migration time at 6.0-8.0 minutes, the perchlorate internal standard migration time at 6.70-9.0 minutes, and the formate major peak at 12-16 minutes (see FIGS. 33A and 33B). The major formate peak, detected in a sample containing 10 µg/ml sodium formate and 3 mg/ml pentosan, should serve as a boundary such that the area defined by the electropherogram curve from the right-most side (i.e., the trailing side) of the formate peak to the right-most end of the broad, bell-shaped pentosan peak is at least 50% of the total area attributed to pentosan. As used herein, the term "Peak Resolution Standard" refers to the use of an internal or reference standard and the other quality controls and methods described in this paragraph.

For optimum resolution of pentosan using CE, the resolution (R) of the sulfate and perchlorate peaks should be at least 2.30 using concentrations of sulfate and perchlorate anions of 50 ug/ml and the United States Pharmacopeia method for calculating resolution in which resolution R=[2(t2-t1)]/[W2+W1], where t2 and W2 are the perchlorate migration time and peak width, and t1 and W1 are the sulfate migration time and peak width, respectively. As used herein, peaks in an electropherogram (e.g., corresponding to perchlorate and sulfate) satisfy the "Optimum Resolution Standard" when the resolution (R) for those peaks is at least 2.30 according the above-described United States Pharmacopeia method for calculating resolution.

In some embodiments, the percentage of the total area of the PPS peak attributable to secondary peaks (e.g., on the leading edge of the PPS peak) may range from 4 to 7%, while the percentage attributable to any single secondary peak may be 1 to 1.5%.

In some embodiments, the percentage of the total area of the PPS peak attributable to secondary peaks (e.g., on the leading edge of the PPS peak) may range from 3 to 12%, while the percentage attributable to any single secondary peak may be 0.5 to 3%.

The homogeneity of a sample of pentosan may be determined by calculating the relative size of small oligosaccharide peaks as a percentage of the total pentosan peak in an electropherogram of a pentosan sample. As used herein, a sample of pentosan that meets a "Pentosan Homogeneity Standard" is a sample for which the discrete peaks detected in an electropherogram on the ascending portion of the pentosan peak (i.e., the portion of the curve attributable to pentosan), excluding the peak due to free sulfate, collectively comprise less than 5% of the total area attributable to pentosan (wherein area is calculated using valley-to-valley integration for individual peaks), and wherein not more than 1.0% of the total pentosan area is contained in any single peak.

FIG. 29 shows an electropherogram of the perchlorate anion in a sample of sodium perchlorate. Two peaks were detected: a major, sharp peak at 12 minutes (291), and a smaller, less sharp peak at approximately 17 minutes (292).

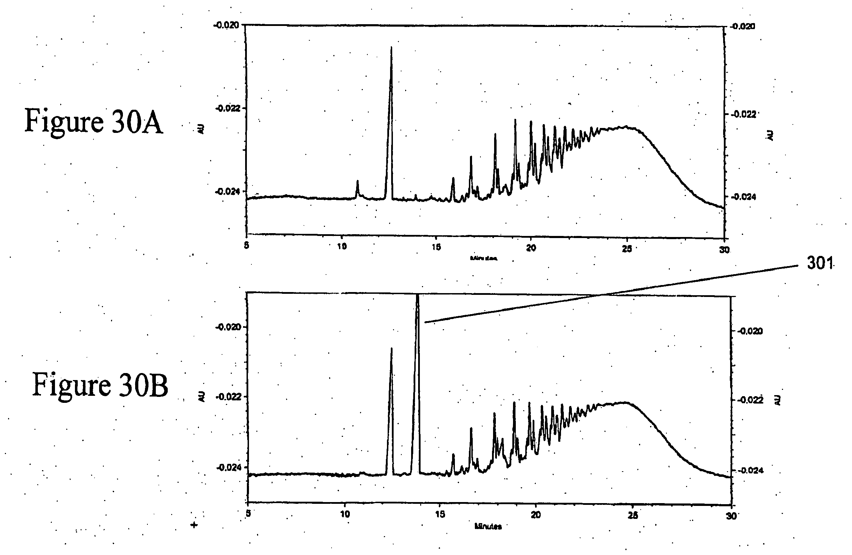

FIGS. 30A and 30B show an electropherogram of a sample of pentosan (Cartrophen^{®}) in the absence (FIG. 30A) and presence of added perchlorate as internal standard (IS) (FIG. 30B). The perchlorate peak migration time appears after the sulfate peak and prior to the appearance of any of the oligosaccharide peaks that characterize the positive control. This migration time makes it suitable as an internal standard that can be added to pentosan samples and used to monitor recovery for quantitation.

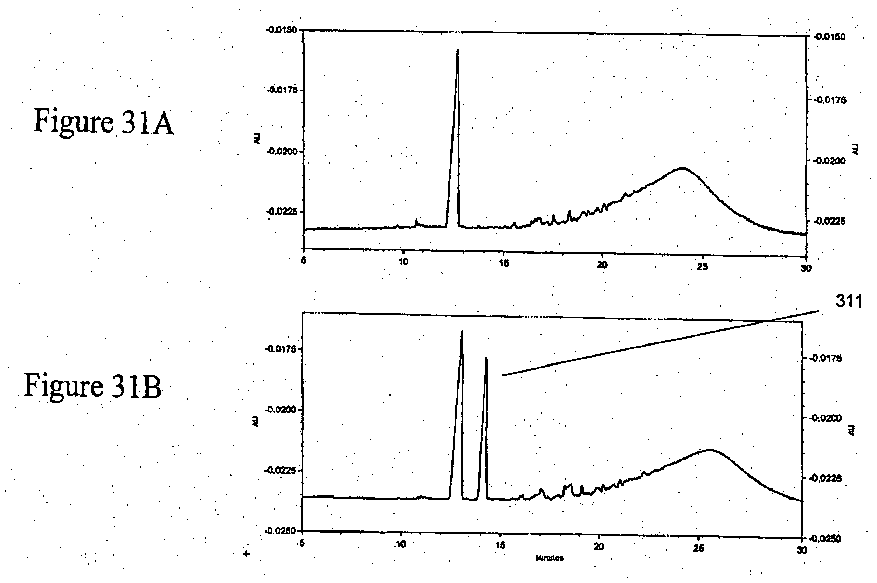

FIGS. 31A and 31B show an electropherogram of pentosan API diluted into water in the absence (FIG. 31A) and presence (FIG. 31B) of added perchlorate used as an internal standard (311). The perchlorate peak appears after the sulfate peak and prior to appearance of any of the minor peaks that precede the broad, substantially homogenous peak of the pentosan.

FIG. 32 shows an electropherogram of the formate anion present in sodium formate. A large, sharp peak appears at 22.5 minutes and a much smaller peak about 29 minutes. The lack of any other peaks and the fact that these migration times appear on both sides of the major pentosan peak makes this anion useful internal reference standard (321) in defining the criteria for acceptable capillary performance for analyzing pentosan samples.

FIGS. 33A and 33B show an electropherogram of pentosan API diluted in water in the absence (FIG. 33A) and presence (FIG. 33B) of added formate. The formate peaks (331) appear on both sides of the main broad peak that characterizes the pentosan. Thus, the formate peaks can serve as markers of its position and relative distance from the sulfate (332) and the perchlorate internal standard peak (333).

While various embodiments of the present invention have been described above, it should be understood that they have been presented by way of example only, and not limitation. Thus, the breadth and scope of the present invention should not be limited by any of the above-described exemplary embodiments, but should be defined only in accordance with the following claims and their equivalents.

## Claims

1. A liquid formulation comprising pentosan polysulfate (PPS) at a concentration of 25 mg/mL to 500 mg/mL and an antioxidant selected from the group consisting of sodium metabisulfite and sodium bisulfite at a concentration of 0.02 % w/v to 5 % w/v of the formulation, wherein the formulation is stable without refrigeration in a solution having a pH in the range of 4 to 8.

2. The formulation of claim 1, further comprising at least one of a chelator, a buffer and an antimicrobial agent.

3. The formulation of claim 2, wherein the antimicrobial agent is selected from the group consisting of methyl paraben, propyl paraben, and benzyl alcohol, and wherein the antimicrobial agent is present in a concentration of 0.05 % w/v to 0.2 % w/v of the formulation.

4. The formulation of claim 1, further comprising EDTA at a concentration of 0.1 mM to 1 mM.

5. The formulation of claim 1, further comprising an aminosugar.

6. The formulation of claim 5, wherein the aminosugar is present at a concentration of 25 mg/mL to 500 mg/mL for injection.

7. The formulation of claims 5 or 6, wherein the aminosugar is selected from the group consisting of glucosamine hydrochloride, galactosamine, glucosamine sulfate, glucosamine phosphate, N-acetyl glucosamine, mannosamine, mixtures or salts thereof.

8. The formulation of claim 1, further comprising hyaluronic acid, preferably in a concentration of 0.1 mg/mL to 50 mg/mL for injection.

9. The formulation of any one of claims 1 to 8, wherein said formulation is an injectable formulation.

10. The formulation of any one of claims 1 to 9, for use as an anticoagulant and/or to treat arthritis.

11. The formulation of any one of claims 1 to 9, for use to treat interstitial cystitis, transmissible spongiform encephalopathy (TSE), immunodeficiency virus, hematomes, hemorrhoids, frostbites, burns, thrombosis, or atherosclerosis.

## Patentansprüche

1. Flüssige Formulierung, umfassend Pentosanpolysulfat (PPS) in einer Konzentration von 25 mg/ml bis 500 mg/ml und ein Antioxidans ausgewählt aus der Gruppe bestehend aus Natriummetabisulfit und Natriumbisulfit in einer Konzentration von 0,02 % w/v bis 5 % w/v der Formulierung, wobei die Formulierung ohne Kühlung in einer Lösung mit einem pH-Wert im Bereich von 4 bis 8 stabil ist.

2. Formulierung nach Anspruch 1, die weiterhin mindestens eines von einem Chelator, einem Puffer und einem antimikrobiellen Mittel umfasst.

3. Formulierung nach Anspruch 2, wobei das antimikrobielle Mittel ausgewählt ist aus der Gruppe bestehend aus Methylparaben, Propylparaben und Benzylalkohol, und wobei das antimikrobielle Mittel in einer Konzentration von 0,05 % w/v bis 0,2 % w/v der Formulierung vorliegt.

4. Formulierung nach Anspruch 1, die weiterhin EDTA in einer Konzentration von 0,1 mM bis 1 mM umfasst.

5. Formulierung nach Anspruch 1, die weiterhin einen Aminozucker umfasst

6. Formulierung nach Anspruch 5, wobei der Aminozucker in einer Konzentration von 25 mg/ml bis 500 mg/ml zur Injektion vorliegt.

7. Formulierung nach Anspruch 5 oder 6, wobei der Aminozucker ausgewählt ist aus der Gruppe bestehend aus Glucosaminhydrochlorid, Galactosamin, Glucosaminsulfat, Glucosaminphosphat, N-Acetylglucosamin, Mannosamin, Mischungen oder Salzen davon.

8. Formulierung nach Anspruch 1, weiter umfassend Hyaluronsäure, vorzugsweise in einer Konzentration von 0,1 mg/ml bis 50 mg/ml zur Injektion.

9. Formulierung nach einem der Ansprüche 1 bis 8, wobei die Formulierung eine injizierbare Formulierung ist.

10. Formulierung nach einem der Ansprüche 1 bis 9, zur Verwendung als Antikoagulans und/oder zur Behandlung von Arthritis.

11. Formulierung nach einem der Ansprüche 1 bis 9 zur Verwendung zur Behandlung von interstitieller Zystitis, übertragbarer spongiformer Enzephalopathie (TSE), Immunschwächevirus, Hämatomen, Hämorrhoiden, Erfrierungen, Verbrennungen, Thrombose oder Atherosklerose.

## Revendications

1. Formulation liquide comprenant du polysulfate de pentosane (PPS) à une concentration de 25 mg/ml à 500 mg/ml et un antioxydant choisi dans le groupe consistant en le métabisulfite de sodium et le bisulfite de sodium à une concentration de 0,02 % p/v à 5 % p/v de la formulation, la formulation étant stable sans réfrigération dans une solution ayant un pH dans la plage de 4 à 8.

2. Formulation selon la revendication 1, comprenant en outre au moins un chélateur, un tampon et un agent antimicrobien.

3. Formulation selon la revendication 2, dans laquelle l'agent antimicrobien est choisi dans le groupe consistant en le méthylparabène, le propylparabène et l'alcool benzylique, et dans laquelle l'agent antimicrobien est présent en une concentration de 0,05 % p/v à 0,2 % p/v de la formulation.

4. Formulation selon la revendication 1, comprenant en outre de l'EDTA en une concentration de 0,1 mM à 1 mM.

5. Formulation selon la revendication 1, comprenant en outre une osamine.

6. Formulation selon la revendication 5, dans laquelle l'osamine est présente en une concentration de 25 mg/ml à 500 mg/ml pour l'injection.

7. Formulation selon les revendications 5 ou 6, dans laquelle l'osamine est choisie dans le groupe consistant en le chlorhydrate de glucosamine, la galactosamine, le sulfate de glucosamine, le phosphate de glucosamine, la N-acétyl glucosamine, la mannosamine, des mélanges ou sels de ceux-ci.

8. Formulation selon la revendication 1, comprenant en outre de l'acide hyaluronique, de préférence en une concentration de 0,1 mg/ml à 50 mg/ml pour l'injection.

9. Formulation selon l'une quelconque des revendications 1 à 8, dans laquelle ladite formulation est une formulation injectable.

10. Formulation selon l'une quelconque des revendications 1 à 9, pour une utilisation en tant qu'anticoagulant et/ou destinée à traiter l'arthrite.

11. Formulation selon l'une quelconque des revendications 1 à 9, pour une utilisation destinée à traiter la cystite interstitielle, l'encéphalopathie spongiforme transmissible (EST), le virus de l'immunodéficience, les hématomes, les hémorroïdes, les engelures, les brûlures, les thromboses ou l'athérosclérose.
